(19) Europäisches Patentamt / European Patent Office / Office européen des brevets

(11) **EP 3 135 633 B1**

(12) # EUROPEAN PATENT SPECIFICATION

(45) Date of publication and mention
of the grant of the patent:
**09.06.2021 Bulletin 2021/23**

(21) Application number: **15786622.9**

(22) Date of filing: **28.04.2015**

(51) Int Cl.:
*C01F 11/20* (2006.01)          *C01G 21/16* (2006.01)
*C01G 23/02* (2006.01)          *H01L 31/042* (2014.01)
*C07C 257/12* (2006.01)          *H01L 51/42* (2006.01)
*H01L 51/00* (2006.01)          *C07C 257/00* (2006.01)

(86) International application number:
**PCT/KR2015/004266**

(87) International publication number:
**WO 2015/167228 (05.11.2015 Gazette 2015/44)**

(54) **PRECURSOR FOR PREPARING PEROVSKITE, PREPARATION METHOD THEREFOR, AND PEROVSKITE SOLAR CELL, AND MANUFACTURING METHOD THEREFOR**

VORLÄUFER ZUR HERSTELLUNG VON PEROWSKIT, HERSTELLUNGSVERFAHREN DAFÜR SOWIE PEROWSKITSOLARZELLE UND HERSTELLUNGSVERFAHREN DAFÜR

PRÉCURSEUR POUR LA PRÉPARATION D'UNE PÉROVSKITE, PROCÉDÉ DE PRÉPARATION ASSOCIÉ, ET CELLULE SOLAIRE EN PÉROVSKITE ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.04.2014 KR 20140051077**
**10.07.2014 KR 20140086580**

(43) Date of publication of application:
**01.03.2017 Bulletin 2017/09**

(73) Proprietors:
• **Research & Business Foundation Sungkyunkwan University Gyeonggi-do 440-746 (KR)**
• **Global Frontier Center for Multiscale Energy Systems Seoul 151-015 (KR)**

(72) Inventors:
• **PARK, Nam-Gyu Seoul 158-759 (KR)**
• **LEE, Jin Wook Yeongyang-gun Gyeongsangbuk-do 764-841 (KR)**
• **SEOL, Dong Jin Seoul 139-752 (KR)**

• **CHO, Anna Gunpo-si Gyeonggi-do 435-722 (KR)**

(74) Representative: **Manitz Finsterwald Patent- und Rechtsanwaltspartnerschaft mbB Martin-Greif-Strasse 1 80336 München (DE)**

(56) References cited:
WO-A1-2014/045021          JP-A- H03 141 247
JP-A- H05 310 756          KR-A- 20140 003 998
US-A1- 2011 087 032

• **Giles E Eperon ET AL: "Supplementary information Formamidinium lead trihalide: a broadly tunable perovskite for efficient planar heterojunction solar cells", , 1 January 2014 (2014-01-01), XP055420379, Retrieved from the Internet: URL:http://www.rsc.org/suppdata/ee/c3/c3ee 43822h/c3ee43822h.pdf [retrieved on 2017-10-31]**
• **ODO K ET AL: "A NEW METHOD FOR THE PREPARATION OF FORMAMIDINE", THE JOURNAL OF ORGANIC CHEMISTRY, AMERICAN CHEMICAL SOCIETY, US, vol. 22, 1 December 1957 (1957-12-01), page 1715, XP000886420, ISSN: 0022-3263, DOI: 10.1021/JO01363A620**

**(Cont. next page)**

- **SHUPING PANG ET AL:** "NH2CH&boxH;NH2Pbl3 : An Alternative Organolead Iodide Perovskite Sensitizer for Mesoscopic Solar Cells", CHEMISTRY OF MATERIALS, vol. 26, no. 3, 11 February 2014 (2014-02-11), pages 1485-1491, XP055234628, ISSN: 0897-4756, DOI: 10.1021/cm404006p
- **CONSTANTINOS C. STOUMPOS ET AL:** "Semiconducting Tin and Lead Iodide Perovskites with Organic Cations: Phase Transitions, High Mobilities, and Near-Infrared Photoluminescent Properties", INORGANIC CHEMISTRY, vol. 52, no. 15, 5 August 2013 (2013-08-05), pages 9019-9038, XP055215452, ISSN: 0020-1669, DOI: 10.1021/ic401215x
- **GILES E. EPERON ET AL.:** 'Formamidinium lead trihalide: a broadly tunable perovskite for efficient planar heterojunction solar cells' ENERGY ENVIRON. SCI. vol. 7, January 2014, pages 9 82 - 988, XP055234624
- **SHUPING PANG ET AL.:** 'NH2 CN =NH2Pb13: An alternative organolead iodide perovskite sensitizer for mesoscopic solar cells' CHEM. MATER. vol. 26, January 2014, pages 1485 - 1491, XP055234628

**Description**

**TECHNICAL FIELD**

The invention relates to a method of preparing a precursor for producing a Perovskite and a method of preparing a Perovskite solar cell using the precursor.

**BACKGROUND**

An organic-inorganic hybrid Perovskite was first applied to a solar cell by Tsutomu Miyasaka's group in 2009 in Japan [J. Am. Chem. Soc. 2009, 131, 6050-6051], and has recently attracted a lot of attention as a light absorbing material for a solar cell due to its high absorption coefficient and property that can be easily synthesized through a solution process.

After the present research team first published a solid hole conductor-based Perovskite solar cell with a photovoltaic conversion efficiency of 9.7% in 2012 [Sci. Rep. 2012, 2:579, 1-7], numerous follow-up studies have been conducted, for example as published by S. Pang, et al. [Chem. Mater., 2014, 26, 1485-1491] or G. E. Eperon, et al. [Energy Environ. Sci., 2014, 7, 982-988]. A formamidinium halide used by S. Pang et al. or G. E. Eperon et al. as a Perovskite precursor for the preparation of the Perovskite solar cell was obtained from formamidinium acetate. Furthermore, a manufacturing method for a Perovskite solar cell and a method for producing a Perovskite using a formamidinium halide obtained from a formamidinium acetate is disclosed in WO 2014/045021 A1. Moreover, a further method for the preparation of a formamidinium iodide from formamidinium chloride is also known from the supporting information of the publication of C. C. Stoumpos et al. in Inorg. Chem., 2013, 52, 9019-9038.

Most of Perovskite photoactive layers researched so far are $CH_3NH_3PbI_3$ and $CH_3NH_3PbI_{3-x}Cl_x$.

The excellent photovoltaic performance of such a methylammonium lead halide Perovskite is derived from its long-range charge transport property and high light absorbing property. The absorption coefficient of $CH_3NH_3PbI_3$ is about one order of magnitude higher than those of molecular sensitizers such as ruthenium bipyridyl complex. Further, $CH_3NH_3PbI_3$ and $CH_3NH_3PbI_{3-x}Cl_x$ can transport electrons and holes when $CH_3NH_3PbI_3$ has diffusion lengths of about 130 nm for an electron and about 100 nm for a hole and $CH_3NH_3PbI_{3-x}Cl_x$ has diffusion lengths of about 1,069 nm for an electron and about 1,213 nm for a hole.

The balanced electron and hole transport properties of $CH_3NH_3PbX_3$ (X=I or I-Cl) form a Perovskite which is a solar cell with a planar heterojunction structure.

Charge accumulation capability of $CH_3NH_3PbI_3$ Perovskite has been reported, and supports generation of free charge carriers and a resultant high open circuit voltage close to about 1.1 V.

Triiodide and the mixed methylammonium lead halide Perovskites are stabilized in a tetragonal phase at room temperature, which have a band gap of about 1.5 eV.

Assuming that there is no loss of incident light caused by reflection from a transparent conductive substrate, a maximum photocurrent density is theoretically about 27.2 mA/cm$^2$ and an absorption onset wavelength measured from an incident photovoltaic conversion efficiency (IPCE) is about 800 nm. However, it is necessary to consider the occurrence of light loss of from about 15% to about 20% in an actual apparatus, and in this case the photocurrent density is from about 23.1 mA/cm$^2$ to about 21.8 mA/cm$^2$. Therefore, a methylammonium lead halide-based Perovskite cell may have a PCE of about 20% when a voltage of about 1.1 V and a fill factor of about 0.8 are satisfied.

However, the methylammonium cation-based organic-inorganic hybrid Perovskite has a band gap of about 1.55 eV and can absorb a light having a wavelength of up to about 800 nm, and thus production amount of photocurrent is limited to about 21 mA/cm$^2$. Further, for a conventional methylammonium cation-based organic-inorganic hybrid Perovskite solar cell, hysteresis is caused by an external voltage, and, thus, it is difficult to precisely measure photovoltaic properties. Furthermore, the conventional methylammonium cation-based organic-inorganic hybrid Perovskite solar cell undergoes a reversible phase transition between tetragonal and cubic symmetry at a temperature between about 300 K and about 400 K corresponding to an operation temperature of the solar cell, and such a structural phase transition is expected to involve a change of a band structure and may affect the photovoltaic properties.

Therefore, a Perovskite which is not affected by a phase transition is demanded. Further, a Perovskite with a low band gap is demanded to further improve a PCE.

A formamidinium cation-based Perovskite was reported as an alternative material to the conventional methylammonium cation-based organic-inorganic hybrid Perovskite solar cell having the above-described problems [Inorg. Chem. 2013, 52, 9019-9038].

However, when the formamidinium cation-based Perovskite is actually applied to a solar cell, it was reported that the maximum efficiency was about 14.2% and an average efficiency was about 9% using a p-i-n junction structure [Energy Environ. Sci., 2014, 7, p982-988].

## DISCLOSURE OF THE INVENTION

### PROBLEMS TO BE SOLVED BY THE INVENTION

Accordingly, the present disclosure provides a method of preparing a precursor for producing a Perovskite and a method of preparing a Perovskite solar cell using the precursor.

However, problems to be solved by the present disclosure are not limited to the above-described problems, and although not described herein, other problems to be solved by the present disclosure can be clearly understood by those skilled in the art from the following descriptions.

### MEANS FOR SOLVING THE PROBLEMS

In accordance with one aspect of the present invention there is provided a method of preparing a precursor for producing a Perovskite, including: reacting formamidine·HCl with sodium ethoxide to obtain a formamidine; and reacting the obtained formamidine with HI to obtain formamidinium iodide.

In accordance with another aspect of the present invention, there is provided a method of preparing a Perovskite solar cell, including: forming a recombination-preventing layer on a first electrode including a transparent conductive substrate; forming a photoactive layer including a Perovskite layer by forming a layer of a Perovskite represented by the following Chemical Formula 1 on the recombination-preventing layer; forming a hole transport layer on the photoactive layer; and forming a second electrode on the hole transport layer:

$$[\text{Chemical Formula 1}] \qquad RMX_3,$$

wherein the forming of a photoactive layer includes forming the layer of the Perovskite represented in Chemical Formula 1 which is formed by reacting $MX_2$ wherein M includes a +2 metal cation selected from the group consisting of $Pb^{2+}$, $Sn^{2+}$, $Ge^{2+}$, and combinations thereof, X is a halogen anion and R being the formamidinium cation of the precursor prepared by said method of preparing a precursor for producing a Perovskite.

### EFFECTS OF THE INVENTION

According to an exemplary embodiment of the present disclosure, a light absorption wavelength region of a Perovskite light-absorbing material can be increased to about 850 nm which is wider by about 50 nm than that of a conventional methylammonium cation-based Perovskite light-absorbing material having a light absorption wavelength region of about 800 nm, by using a formamidinium cation-based Perovskite having a band gap energy of about 1.45 eV reduced by about 0.1 eV as compared with the conventional methylammonium cation-based Perovskite.

According to an exemplary embodiment of the present disclosure, when preparing a precursor for producing a Perovskite, a formamidinium cation and a metal alkoxide including an alkali metal are used, and, thus, it is easy to remove by-products and the precursor for producing a Perovskite can be prepared with a high yield of about 90% or more, as compared with a conventional precursor for producing a Perovskite. In particular, if sodium ethoxide is used as the metal alkoxide, it is easy to remove by-products, i.e., NaX (e.g., NaCl), $C_2H_5OH$, and the like after the reaction and thus the yield of the precursor can be improved.

According to an exemplary embodiment of the present disclosure, the efficiency of a solar cell is generally determined by multiplying a photocurrent, a photovoltage, and a fill factor, and since the amount of photocurrent is increased according to an exemplary embodiment of the present disclosure, improvement in final efficiency of a solar cell can be expected.

Further, according to an exemplary embodiment of the present disclosure, the occurrence of hysteresis caused by an external voltage is decreased as compared with a conventional methylammonium cation-based Perovskite solar cell, and, thus, it is possible to easily measure photovoltaic properties.

### BRIEF DESCRIPTION OF THE DRAWINGS

FIG. 1A to FIG. 1C illustrate a structure of a Perovskite solar cell in accordance with an exemplary embodiment of the present disclosure.

FIG. 2A to FIG. 2C are images of precursor powder for Perovskite solar cell obtained in accordance with an example of the present disclosure.

FIG. 3 is a H NMR spectrum of a precursor for solar cell prepared by a synthesis method in accordance with an example of the present disclosure.

**FIG. 4** is a H NMR spectrum of a precursor for solar cell prepared by a synthesis method in accordance with Comparative Example 1 of the present disclosure.

**FIG. 5** is a H NMR spectrum of a precursor for solar cell prepared by a synthesis method in accordance with Comparative Example 2 of the present disclosure.

**FIG. 6A** to **FIG. 6F** show light absorption properties of a Perovskite solar cell in accordance with an example of the present disclosure.

**FIG. 7A** to **FIG. 7E** show X-ray diffraction patterns depending on a temperature in accordance with an example of the present disclosure.

**FIG. 8A** to **FIG. 8F** show photovoltaic effects depending on an annealing duration at about 150°C in accordance with an example of the present disclosure.

**FIG. 9A** to **FIG. 9E** show photovoltaic effects depending on a thickness of a TiO$_2$-containing semiconductor layer in accordance with an example of the present disclosure.

**FIG. 10A** to **FIG. 10F** are SEM images showing an upper part of a photoactive layer formed on a TiO$_2$-containing semiconductor layer in accordance with an example of the present disclosure.

**FIG. 11A** and **FIG. 11B** show the dependence of IPCE depending on a thickness of a TiO$_2$-containing semiconductor layer in accordance with an example of the present disclosure.

**FIG.12A** to **FIG. 12F** show effects depending on a content of a polymer present in TiO$_2$ paste in accordance with an example of the present disclosure.

**FIG. 13** shows an effect depending on a content of a polymer present in TiO$_2$ paste in accordance with an example of the present disclosure.

**FIG. 14** shows a photocurrent-voltage curve of a solar cell including HC(NH$_2$)$_2$PbI$_3$ Perovskite in accordance with an example of the present disclosure.

## BEST MODE FOR CARRYING OUT THE INVENTION

Hereinafter, embodiments and examples of the present disclosure will be described in detail with reference to the accompanying drawings so that the present disclosure may be readily implemented by those skilled in the art. However, it is to be noted that the present disclosure is not limited to the embodiments and examples but can be embodied in various other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element.

Through the whole document, the term "on" that is used to designate a position of one element with respect to another element includes both a case that the one element is adjacent to the another element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise. Through the whole document, the term "about or approximately" or "substantially" are intended to have meanings close to numerical values or ranges specified with an allowable error and intended to prevent accurate or absolute numerical values disclosed for understanding of the present disclosure from being illegally or unfairly used by any unconscionable third party. Through the whole document, the term "step of" does not mean "step for".

Through the whole document, the term "combination of" included in Markush type description means mixture or combination of one or more components, steps, operations and/or elements selected from a group consisting of components, steps, operation and/or elements described in Markush type and thereby means that the disclosure includes one or more components, steps, operations and/or elements selected from the Markush group.

Through the whole document, a phrase in the form "A and/or B" means "A or B, or A and B".

Through the whole document, a "Perovskite" has an ABX$_3$ structure, and the ABX$_3$ structure can be readily estimated based on a tolerance factor t:

$$t = (r_A + r_X)/[2^{1/2}(r_B + r_X)]$$

Herein, r$_A$ and r$_B$ are effective ionic radiuses of cations in a cubo-octahedral A-site and an octahedral B-site, respectively, and r$_X$ is a radius of an anion.

Hereinafter, exemplary embodiments and examples of the present disclosure will be described in detail with reference to the accompanying drawings. However, the present disclosure may not be limited to the exemplary embodiment, examples, and drawings.

In accordance with a first aspect of the present disclosure, there is provided a method of preparing a precursor for producing a Perovskite, including: reacting a formamidine·HX wherein X is a halide with a metal alkoxide to obtain a formamidine; and reacting the obtained formamidine with HX wherein X is a halide to obtain a a formamidinium halide.

In accordance with an exemplary embodiment of the present disclosure, the metal alkoxide may include $MOR_1$, wherein M is a +2 alkali metal cation and $R_1$ includes a +1 cation selected from the group consisting of $C_nH_{2n+1}NH_3^+$ wherein n is an integer of from 1 to 9, $NH_4^+$, $HC(NH_2)_2^+$, $CS^+$, $NF_4^+$, $NCl_4^+$, $PF_4^+$, $PCl_4^+$, $CH_3PH_3^+$, $CH_3ASH_3^+$, $CH_3SbH_3^+$, $PH_4^+$, $ASH_4^+$, $SbH_4^+$, and combinations thereof.

In accordance with an exemplary embodiment of the present disclosure, $R_1$ may include a linear or branched, saturated or unsaturated alkyl group having 1 to 20 carbon atoms, and may include, for example, methyl, ethyl, propyl, butyl, pentyl, hexyl, heptyl, octyl, nonyl, decyl, or all available isomers thereof, but may not be limited thereto.

In accordance with an exemplary embodiment of the present disclosure, the metal alkoxide may include an alkali metal alkoxide, but may not be limited thereto. For example, the metal alkoxide may include sodium ethoxide, but may not be limited thereto. In accordance with an exemplary embodiment of the present disclosure, if a precursor for producing a Perovskite is prepared using a metal alkoxide, it is easy to remove by-products generated during a process for preparing the Perovskite precursor and it is possible to obtain the precursor for producing a Perovskite with a high yield.

In accordance with an exemplary embodiment of the present disclosure, the HX may include a member selected from the group consisting of HI, HCl, HBr, and combinations thereof, but may not be limited thereto.

In accordance with an exemplary embodiment of the present disclosure, the formamidinium halide may include a member selected from the group consisting of formamidinium iodide, formamidinium chloride, formamidinium bromide, and combinations thereof, but may not be limited thereto.

A solar cell may be prepared using a Perovskite prepared using the precursor for producing a Perovskite acquired by the method in accordance with the first aspect of the present disclosure, and, thus, it is possible to prepare a Perovskite solar cell with an excellent photovoltaic efficiency.

In accordance with a second aspect of the present disclosure, there is provided a precursor for producing a Perovskite including a formamidinium halide prepared by the method in accordance with the first aspect of the present disclosure.

In accordance with an exemplary embodiment of the present disclosure, the formamidinium halide may include an alkali metal, but may not be limited thereto.

In accordance with a third aspect of the present disclosure, there is provided a Perovskite solar cell including: a first electrode including a transparent conductive substrate; a recombination-preventing layer formed on the first electrode; a photoactive layer formed on the recombination-preventing layer; a hole transport layer formed on the photoactive layer; and a second electrode formed on the hole transport layer, and the photoactive layer includes a Perovskite layer including a Perovskite represented by the following Chemical Formula 1:

$$[\text{Chemical Formula 1}] \qquad RMX_3,$$

in Chemical Formula 1, R includes a +1 cation selected from the group consisting of $C_nH_{2n+1}NH_3^+$ wherein n is an integer of from 1 to 9, $NH_4^+$, $HC(NH_2)_2^+$, $CS^+$, $NF_4^+$, $NCl_4^+$, $PF_4^+$, $PCl_4^+$, $CH_3PH_3^+$, $CH_3ASH_3^+$, $CH_3SbH_3^+$, $PH_4^+$, $ASH_4^+$, $SbH_4^+$, and combinations thereof, M includes a +2 metal cation selected from the group consisting of $Pb^{2+}$, $Sn^{2+}$, $Ge^{2+}$, and combinations thereof, and X is a halogen anion.

In accordance with an exemplary embodiment of the present disclosure, in Chemical Formula 1, R may include a linear or branched, saturated or unsaturated alkyl group having 1 to 20 carbon atoms, and may include, for example, methyl, ethyl, propyl, butyl, pentyl, hexyl, heptyl, octyl, nonyl, decyl, or all available isomers thereof, but may not be limited thereto.

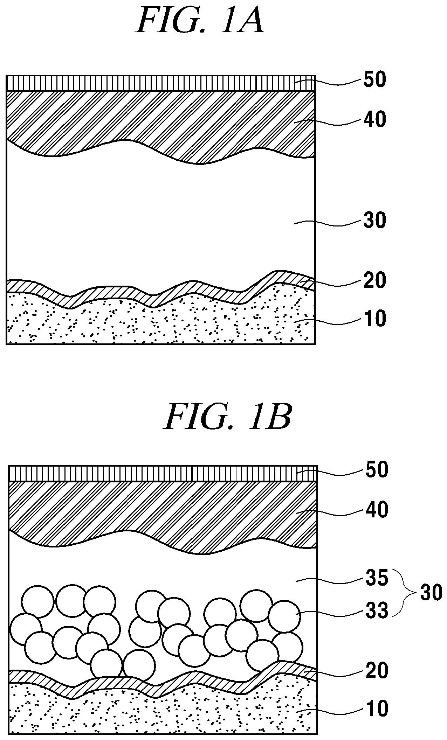

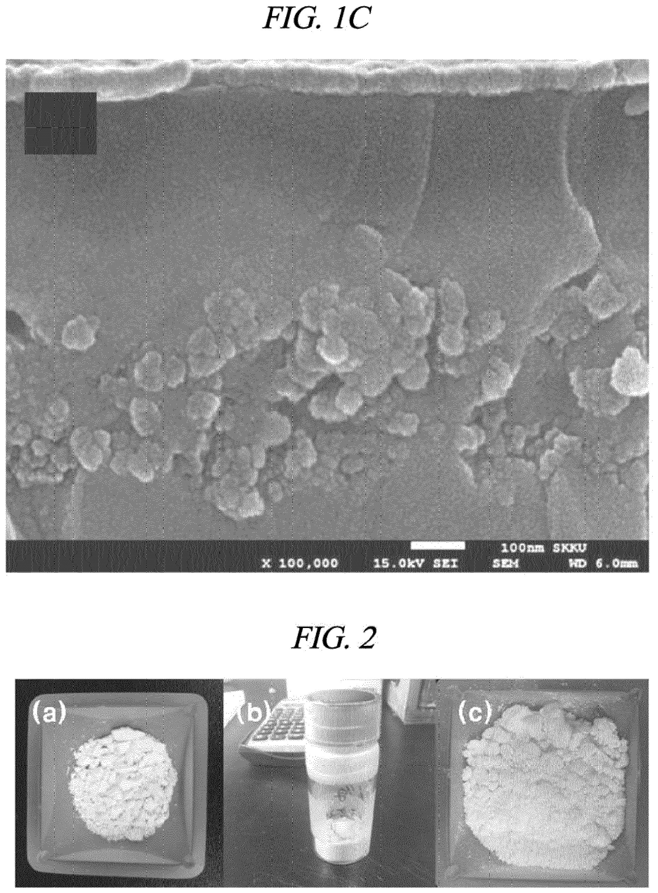

In this regard, **FIG. 1A** and **FIG. 1B** are schematic diagrams illustrating a layer structure of a Perovskite solar cell in accordance with an exemplary embodiment of the present disclosure, and **FIG. 1C** is a SEM image capturing a layer structure of a Perovskite solar cell prepared in accordance with an example of the present disclosure.

Referring to **FIG. 1A,** a Perovskite solar cell of the present disclosure may have a sandwich structure in which two electrodes, i.e., a first electrode 10 and a second electrode 20, are in surface contact with each other, but may not be limited thereto. For example, the first electrode 10 may be represented as a working electrode or a semiconductor electrode, and the second electrode 50 may be represented as a counter electrode, but may not be limited thereto.

A recombination-preventing layer 20 is formed on the first electrode, and a photoactive layer 30 is formed on the recombination-preventing layer 20 and may include a layer of a Perovskite represented by the following Chemical Formula 1.

[Chemical Formula 1]          $RMX_3$,

in Chemical Formula 1, R includes a +1 cation selected from the group consisting of $C_nH_{2n+1}NH_3^+$ wherein n is an integer of from 1 to 9, $NH_4^+$, $HC(NH_2)_2^+$, $CS^+$, $NF_4^+$, $NCl_4^+$, $PF_4^+$, $PCl_4^+$, $CH_3PH_3^+$, $CH_3ASH_3^+$, $CH_3SbH_3^+$, $PH_4^+$, $ASH_4^+$, $SbH_4^+$, and combinations thereof, M includes a +2 metal cation selected from the group consisting of $Pb^{2+}$, $Sn^{2+}$, $Ge^{2+}$, and combinations thereof, and X is a halogen anion.

In accordance with an exemplary embodiment of the present disclosure, the layer of a Perovskite may include the Perovskite represented by Chemical Formula 1 and formed by reacting $MX_2$ (herein, M includes a +2 metal cation selected from the group consisting of $Pb^{2+}$, $Sn^{2+}$, $Ge^{2+}$, and combinations thereof and X is a halogen anion) with the precursor for producing a Perovskite including formamidinium halide in accordance with the second aspect of the present disclosure, but may not be limited thereto. The precursor for producing a Perovskite including a formamidinium halide may be prepared by reacting, particularly, a formamidinium cation with a metal alkoxide $MOR_1$ (herein, M is a +2 metal cation and $R_1$ includes a +1 cation selected from the group consisting of $C_nH_{2n+1}NH_3^+$ wherein n is an integer of from 1 to 9, $NH_4^+$, $HC(NH_2)_2^+$, $CS^+$, $NF_4^+$, $NCl_4^+$, $PF_4^+$, $PCl_4^+$, $CH_3PH_3^+$, $CH_3ASH_3^+$, $CH_3SbH_3^+$, $PH_4^+$, $ASH_4^+$, $SbH_4^+$, and combinations thereof), but may not be limited thereto. For example, the metal alkoxide may include sodium ethoxide, but may not be limited thereto. If a precursor for producing a Perovskite is prepared using the metal alkoxide $MOR_1$, it is easy to remove by-products generated during a process for preparing the Perovskite precursor and it is possible to acquire the precursor for producing a Perovskite with a high yield.

In accordance with an exemplary embodiment of the present disclosure, the Perovskite in Chemical Formula 1 may include $HC(NH_2)_2MX_3$, but may not be limited thereto.

In accordance with an exemplary embodiment of the present disclosure, the layer of a Perovskite may have a thickness of about 1 μm or less, but may not be limited thereto. For example, the layer of a Perovskite may have a thickness of about 1 μm or less, about 900 nm or less, about 800 nm or less, about 700 nm or less, about 600 nm or less, about 500 nm or less, about 400 nm or less, about 300 nm or less, about 200 nm or less, or about 100 nm or less, and desirably about 300 nm or less, but may not be limited thereto.

In accordance with an exemplary embodiment of the present disclosure, the layer of a Perovskite may include a layer 35 of Perovskite $HC(NH_2)_2MX_3$ prepared by reacting $MX_2$ with a precursor $HC(NH_2)_2I$ prepared by the method in accordance with the first aspect of the present disclosure.

In accordance with an exemplary embodiment of the present disclosure, the Perovskite $RMX_3$ structure can be readily estimated based on a tolerance factor t: $t = (r_R + r_X)/[2^{1/2}(r_M + r_X)]$. Herein, $r_R$ and $r_M$ are effective ionic radii of the cations in a cubo-octahedral R-site and an octahedral M-site, respectively, and $r_X$ is a radius of the anion. A radius of the cation from $RPbI_3$ was calculated in the range of from 164 pm for t = 0.8 to 259 pm for t = 1 based on $Pb^{2+}$ = 119 pm and $I^-$ = 220 pm. For example, $CH_3NH_3^+$ cation has an ionic radius of 180 pm and thus is suitable for a Perovskite structure. A change in the cation may result in a change in Pb-I bond length and/or bond angle, which causes a change in band structure and band gap tuning. Substitution by slightly larger formamidinium $[HC(NH_2)_2^+]$ for methylammonium $(CH_3NH_3^+)$ can be stabilized as a trigonal Perovskite structure (black) or a hexagonal non-Perovskite structure (yellow). A band gap of $HC(NH_2)_2PbI_3$ was estimated at 1.45 eV which is lower than that(1.52 eV) of $CH_3NH_3PbI_3$ due to the lowered symmetry. Therefore, it is expected that $HC(NH_2)_2PbI_3$ absorbs light in a wider range than $CH_3NH_3PbI_3$ due to its lower band gap, and, thus, it is expected that $HC(NH_2)_2PbI_3$ has a higher PCE due to a higher photocurrent. Further, a remarkable phase transition in an operation temperature range of a solar cell is not expected. According to a conventional study, it was found that dry crystals (a-phase) of black $HC(NH_2)_2PbI_3$ are converted into β-phase at about 200 K or less and converted into γ-phase at about 130 K or less. However, $HC(NH_2)_2PbI_3$ in a Perovskite solar cell showed a low PCE of 4.3% with an unexpectedly very low photocurrent density of 6.45 mA/cm², and in this case, the low photocurrent can be explained as being caused by non-uniformity in conduction band edge between $TiO_2$ and $FAPbI_3$. Since it was confirmed that a scaffold layer including an electron non-injected oxide such as $Al_2O_3$ or $ZrO_2$ is easily operated in a Perovskite solar cell, it may not be a reason for explaining the low photocurrent.

A hole transport layer (hereinafter, also referred to as "HTM") 40 is formed on the photoactive layer 30, and the second electrode 50 may be formed on the hole transport layer 40. The hole transport layer 40 may be formed in order to reduce the oxidized photoactive layer 30, but may not be limited thereto.

In accordance with an exemplary embodiment of the present disclosure, the transparent conductive substrate may include a glass substrate containing a material selected from the group consisting of indium tin oxide (ITO), fluorine tin oxide (FTO), $ZnO-Ga_2O_3$, $ZnO-Al_2O_3$, tin-based oxide, zinc oxide, and combinations thereof or a plastic substrate, but may not be limited thereto. The transparent conductive substrate may use any material without particular limitations as long as it has conductance and transparency. For example, the plastic substrate may include a member selected from the group consisting of polyethylene terephthalate, polyethylene naphthalate, polycarbonate, polypropylene, polyimide, triacetyl cellulose, and combinations thereof, but may not be limited thereto.

In accordance with an exemplary embodiment of the present disclosure, the photoactive layer 30 may further include a semiconductor layer 33 as an electron transport layer, as illustrated in **FIG. 1B,** but may not be limited thereto.

For example, after the semiconductor layer 33 is formed on the electron recombination-preventing layer 20, the Perovskite layer 35 may be formed on the semiconductor layer 33, but may not be limited thereto.

In accordance with an exemplary embodiment of the present disclosure, the semiconductor layer 33 may have a thickness of about 1 μm or less, but may not be limited thereto. For example, the semiconductor layer 33 may have a thickness of about 1 μm or less, about 900 nm or less, about 800 nm or less, about 700 nm or less, about 600 nm or less, about 500 nm or less, about 400 nm or less, about 300 nm or less, about 200 nm or less, or about 100 nm or less, and desirably about 300 nm or less, but may not be limited thereto. In accordance with an exemplary embodiment of the present disclosure, the semiconductor layer 33 may have a thickness equal to or smaller than that of the Perovskite layer 35. For example, the semiconductor layer 33 may be included in the Perovskite layer 35 as illustrated in **FIG. 1B,** or the Perovskite infiltrates into pores of the semiconductor layer having a mesoporous structure.

In accordance with an exemplary embodiment of the present disclosure, the semiconductor layer 33 may have a mesoporous structure including pores of from about 20 nm to about 500 nm, but may not be limited thereto. For example, the semiconductor layer 33 may include pores of from about 20 nm to about 500 nm, from about 20 nm to about 450 nm, from about 20 nm to about 400 nm, from about 20 nm to about 350 nm, from about 20 nm to about 300 nm, from about 20 nm to about 250 nm, from about 20 nm to about 200 nm, from about 20 nm to about 150 nm, from about 20 nm to about 100 nm, or from about 20 nm to about 50 nm, but may not be limited thereto. For example, the pores of the semiconductor layer may be formed using a polymer as a sacrificial material for forming pores. The polymer sacrificial material may include a polymer selected from the group consisting of ethylene cellulose, hydroxypropylcellulose, polyethyleneglycol, polyethyleneoxide, polyvinylalcohol, polyvinylpyrrolidone, and combinations thereof, but may not be limited thereto.

In accordance with an exemplary embodiment of the present disclosure, the semiconductor layer may include an organic semiconductor, an inorganic semiconductor, or mixture thereof, but may not be limited thereto.

In accordance with an exemplary embodiment of the present disclosure, the semiconductor layer may include a metal oxide selected from the group consisting of $TiO_2$, $SnO_2$, ZnO, $WO_3$, $Nb_2O_5$, $TiSrO_3$, and combinations thereof, but may not be limited thereto.

In accordance with an exemplary embodiment of the present disclosure, the hole transport layer 40 may include a unimolecular or polymeric hole transport material, but may not be limited thereto. For example, spiro-MeOTAD [2,2',7,7'-tetrakis(N,N-p-dimethoxy-phenylamino)-9,9'-spirobifluorene] may be used as the unimolecular hole transport material and P3HT[poly(3-hexylthiophene)] may be used as the polymeric hole transport material, but may not be limited thereto. Further, for example, the hole transport layer 40 may use a dopant selected from the group consisting of Li-based dopants, Co-based dopants, and combinations thereof, but may not be limited thereto. For example, a mixture of spiro-MeOTAD, tBP, and Li-TFSI may be used as the hole transport material, but the present disclosure may not be limited thereto.

In accordance with an exemplary embodiment of the present disclosure, the second electrode may include a member selected from the group consisting of Pt, Au, Ni, Cu, Ag, In, Ru, Pd, Rh, Ir, Os, C, conductive polymers, and combinations thereof, but may not be limited thereto. For example, highly stable gold (Au) may be used as the second electrode to improve the long-term stability of the Perovskite solar cell of the present disclosure, but the present disclosure may not be limited thereto.

In accordance with a fourth aspect of the present disclosure, there is provided a method of preparing a Perovskite solar cell, including: forming a recombination-preventing layer on a first electrode including a transparent conductive substrate; forming a photoactive layer including a Perovskite layer by forming a layer of a Perovskite represented by the following Chemical Formula 1 on the recombination-preventing layer; forming a hole transport layer on the photoactive layer; and forming a second electrode on the hole transport layer:

[Chemical Formula 1]   $RMX_3$,

in Chemical Formula 1, R includes a +1 cation selected from the group consisting of $C_nH_{2n+1}NH_3^+$ wherein n is an integer of from 1 to 9, $NH_4^+$, $HC(NH_2)_2^+$, $CS^+$, $NF_4^+$, $NCl_4^+$, $PF_4^+$, $PCl_4^+$, $CH_3PH_3^+$, $CH_3ASH_3^+$, $CH_3SbH_3^+$, $PH_4^+$, $ASH_4^+$, $SbH_4^+$, and combinations thereof, M includes a +2 metal cation selected from the group consisting of $Pb^{2+}$, $Sn^{2+}$, $Ge^{2+}$, and combinations thereof, and X is a halogen anion.

Since the fourth aspect of the present disclosure relates to a method of preparing the Perovskite solar cell in accordance with the third aspect of the present disclosure, detailed descriptions of the method of preparing the Perovskite solar cell, which overlap with those of the third aspect of the present disclosure, are omitted hereinafter, but the descriptions of the third aspect of the present disclosure may be identically applied to the fourth aspect of the present disclosure, even though they are omitted hereinafter.

In accordance with an exemplary embodiment of the present disclosure, the forming of a photoactive layer may include forming the layer of the Perovskite represented by Chemical Formula 1 formed by reacting $MX_2$ (herein, M includes a +2 metal cation selected from the group consisting of $Pb^{2+}$, $Sn^{2+}$, $Ge^{2+}$, and combinations thereof and X is a halogen anion) with the precursor for producing a Perovskite including formamidinium halide in accordance with the second aspect of the present disclosure, but may not be limited thereto.

In accordance with an exemplary embodiment of the present disclosure, the photoactive layer may further include a semiconductor layer, but may not be limited thereto.

In accordance with an exemplary embodiment of the present disclosure, the semiconductor layer may have a thickness of about 1 $\mu$m or less, but may not be limited thereto. For example, the semiconductor layer may have a thickness of about 1 $\mu$m or less, about 900 nm or less, about 800 nm or less, about 700 nm or less, about 600 nm or less, about 500 nm or less, about 400 nm or less, about 300 nm or less, about 200 nm or less, or about 100 nm or less, and desirably about 300 nm or less, but may not be limited thereto.

In accordance with an exemplary embodiment of the present disclosure, the semiconductor layer may have a mesoporous structure including pores of from about 20 nm to about 500 nm, but may not be limited thereto. For example, the semiconductor layer may include pores of from about 20 nm to about 500 nm, from about 20 nm to about 450 nm, from about 20 nm to about 400 nm, from about 20 nm to about 350 nm, from about 20 nm to about 300 nm, from about 20 nm to about 250 nm, from about 20 nm to about 200 nm, from about 20 nm to about 150 nm, from about 20 nm to about 100 nm, or from about 20 nm to about 50 nm, but may not be limited thereto.

In accordance with an exemplary embodiment of the present disclosure, the semiconductor layer may include an organic semiconductor, an inorganic semiconductor, or mixture thereof, but may not be limited thereto.

In accordance with an exemplary embodiment of the present disclosure, the semiconductor layer having the mesoporous structure may be formed by forming and coating a mixture including metal oxide semiconductor nanoparticles and a polymer as a sacrificial material for forming pores and then forming pores by removing the polymer, but may not be limited thereto. In accordance with an exemplary embodiment of the present disclosure, a pore size of the semiconductor layer may be regulated by regulating a content of the polymer included in the semiconductor layer, but may not be limited thereto.

In accordance with an exemplary embodiment of the present disclosure, the method may further include annealing the photoactive layer at from 100°C to 170°C right after the photoactive layer is formed, but may not be limited thereto. For example, the annealing may be performed at from about 100°C to about 170°C, from about 100°C to about 160°C, from about 100°C to about 150°C, from about 100°C to about 140°C, from about 100°C to about 130°C, from about 100°C to about 120°C, from about 100°C to about 110°C, from about 110°C to about 170°C, from about 110°C to about 160°C, from about 110°C to about 150°C, from about 110°C to about 140°C, from about 110°C to about 130°C, from about 110°C to about 120°C, from about 120°C to about 170°C, from about 120°C to about 160°C, from about 120°C to about 150°C, from about 120°C to about 140°C, from about 120°C to about 130°C, from about 130°C to about 170°C, from about 130°C to about 160°C, from about 130°C to about 150°C, from about 130°C to about 140°C, from about 140°C to about 170°C, from about 140°C to about 160°C, from about 140°C to about 150°C, from about 150°C to about 170°C, from about 150°C to about 160°C, or from about 160°C to about 170°C, but may not be limited thereto.

## MODE FOR CARRYING OUT THE INVENTION

Hereinafter, examples of the present disclosure will be described in more detail. However, the scope of the present disclosure may not be limited thereto.

**[Example]**

### 1. Preparation of precursor $HC(NH_2)_2I$ for Perovskite

In order to prepare formamidinium iodide [$HC(NH_2)_2I$] as a precursor for Perovskite solar cell in accordance with the first aspect of the present disclosure, 0.1 mol NaH (TCI, 60 wt.%) was put into 100 mL ethanol solution (Samchun, 99.99%) to synthesize 0.1 mol sodium ethoxide solution, as shown in the following Reaction Formula 1:

$$NaH + C_2H_5OH \rightarrow NaOC_2H_5 + H_2 (\uparrow) \qquad (1).$$

Then, the sodium ethoxide solution was put into a round flask and 0.1 mol HC(NH)(NH$_2$). HCl (TCI, >95%) was added thereto with stirring, as shown in the following Reaction Formula 2:

$$HC(NH)(NH_2). HCl + NaOC_2H_5 \rightarrow HC(NH)(NH_2) + NaCl (\downarrow) + C_2H_5OH \qquad (2).$$

Then, a precipitate NaCl as a by-product was filtered using a filter paper. $C_2H_5OH$ as another by-product was easily removed by evaporation. A solution obtained after the filtering was put into a round flask and a bath was filled with ice and water, and then the round flask was fixed in the bath such that at least half of the flask was submerged therein.

Finally, 0.1 mol hydroiodic acid (HI) (Aldrich, 57 wt.% in water) was added in a dropwise manner to the solution with stirring, as shown in the following Reaction Formula 3:

$$HC(NH)(NH_2) + HI \rightarrow HC(NH_2)_2I \qquad (3).$$

After the dropping of the HI, a reaction was carried out for 2 hours, and all the solvent was removed at 60°C using a rotary evaporator.

In a state where all the solvent was removed, diethyl ether was added thereto such that all the product was submerged therein, and after stirring for 20 minutes, a process of removing the solution was repeated 5 times.

After the washing process, $HC(NH_2)_2I$ was filtered and then dried in a vacuum for 12 hours, so that a precursor $HC(NH_2)_2I$ to form white powder as shown in **FIG. 2A.**

The method of synthesizing a precursor $HC(NH_2)_2I$ in accordance with the present Example is different from a conventional method using HI and formamidine acetate in which acetic acid as a by-product is not easily removed from $HC(NH_2)_2I$. Since the precipitate NaCl in Reaction Formula 2 has a much lower solubility in ethanol, ethanol was used instead of methanol in the present Example (0.65 g NaCl/1 kg of ethanol vs 14 g NaCl/1 kg of methanol).

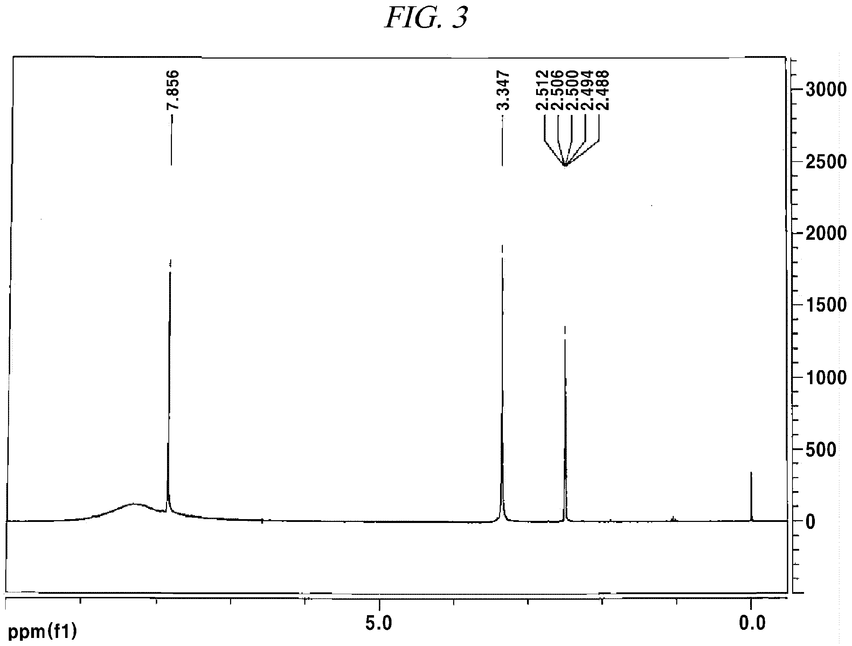

The purity of the precursor $HC(NH_2)_2I$ synthesized according to the present Example was confirmed by $^1$H NMR and high resolution mass spectroscopy as shown in **FIG. 3.**

The yield of the $HC(NH_2)_2I$ was 90% or more.

$^1$H NMR (300 MHz, DMSO) d 7.857 (s, 1H), 8.314 (br, 4H).

The high resolution mass spectroscopy (HRMS)(FAB) calculated for $CH_5IN_2$ Na $[M+Na]^+$: 194.9390, found: 194.9395.

2. Preparation of Perovskite $HC(NH_2)_2PbI_3$ solar cell

In order to prepare the Perovskite solar cell in accordance with the third aspect of the present disclosure, the first electrode including the transparent conductive substrate was first produced, and the recombination-preventing layer was formed on the first electrode, and a porous semiconductor/Perovskite complex layer including the porous semiconductor layer and the precursor $HC(NH_2)_2I$ prepared as shown in Reaction Formula 3 was formed on the recombination-preventing layer.

First, a fluorine-doped tin oxide-coated (FTO) glass (Pilkington, TEC-8, 8 Ω/sq) was washed by UV-ozone treatment for 15 minutes and consecutively washed with detergent and ethanol.

A dense $TiO_2$ blocking layer (BL) as the recombination-preventing layer was formed by spin-coating a 0.15 M titanium diisopropoxide bis(acetylacetonate (Aldrich, 75 wt.% in isopropanol)/1-butanol (Aldrich, 99.8%) solution and annealing at 500°C for 15 minutes.

A porous $TiO_2$ film was deposited on the dense $TiO_2$ BL by spin-coating $TiO_2$ paste diluted in ethanol.

The $TiO_2$ paste was prepared by mixing hydrothermally synthesized $TiO_2$ nanoparticles of 50 nm, ethyl cellulose (EC) in terpineol (TP), and lauric acid (LA) (nominal ratio of $TiO_2$:TP:EC:LA = 1.25:6:0.6~1.5:0.1).

The content of the EC was varied from 0.6 to 1.5 in order to control a pore size and porosity.

The content of the paste was varied from 0.8 g to 2 g in 10 ml ethanol in order to control the thickness of the film.

0.8 g of the paste in 10 mL ethanol resulted in 130 nm, and 2 g of the paste resulted in 320 nm.

The rate of increase in thickness was about 160 nm/g $TiO_2$.

The $TiO_2$ paste-coated electrode was annealed at 550°C for 1 hour.

The electrode was cooled to room temperature and then immersed in a 20 mM $TiCl_4$ (Aldrich, >98%) aqueous solution at 70°C for 10 minutes and washed with deionized water and then annealed at 500°C for 30.

$HC(NH_2)_2PbI_3$ having a thickness of 300 nm was formed on the porous $TiO_2$ film by a two-step method using the formamidinium iodide and $PbI_2$ available in the market.

4 mmol (1.844 g) $PbI_2$ (Aldrich, 99%) was dissolved in 4 mL N,N-dimethylformamide (DMF) (Sigma-Aldrich, 99.8%) with stirring at 70°C.

30 μL of the $PbI_2$ solution was rotated on the porous $TiO_2$ film at 500 rpm for 5 seconds and at 6,000 rpm for 20 seconds.

The $PbI_2$-coated $TiO_2$ film was dried at 40°C for 3 minutes and at 100°C for 10 minutes.

In order to prepare $HC(NH_2)_2PbI_3$, the $PbI_2$-coated $TiO_2$ film was immersed for 1 minute in a 0.01 g/mL $HC(NH_2)_2I$ solution in 2-propanol (Sigma-Aldrich, 99.5%) and rotated at 500 rpm for 5 seconds, at 1,500 rpm for 10 seconds, and at 3,000 rpm for 20 seconds, and then dried at 40°C for 3 minutes and at 150 °C for 15 minutes.

After the immersion in the $HC(NH_2)_2I$, a rotation process was performed in order to uniformly dry the film.

In order to remove the overabundant $HC(NH_2)_2I$ which was not reacted, the film was immersed for 15 minutes and washed with the 2-propanol solution and then dried at 150°C for 5 minutes.

A solution for spiro-MeOTAD coating was prepared by dissolving 72.3 mg spiro-MeOTAD in 1 mL chloroben-

zene, and 28.8 μL 4-tert-butyl pyridine and 17.5 μL lithium bis(trifluoromethanesulfonyl)imide] solution [520 mg Li-TFSI in 1 mL acetonitrile (Sigma-Aldrich, 99.8%)] were added thereto.

The spiro-MeOTAD was deposited at 4,000 rpm for 20 seconds.

Finally, as a counter electrode, gold was thermally evaporated at an evaporation rate of 1 Å/s.

**[Comparative Example 1]**

Preparation of precursor $HC(NH_2)_2I$

A precursor $HC(NH_2)_2I$ was prepared using $HC(NH)(NH_2)$, HCl and methanol as shown in the following Formula 4:

$$HC(NH)(NH_2). \; HCl + NaOCH_3 \rightarrow HC(NH)(NH_2) + NaCl \; (\downarrow) + CH_3OH \qquad (4).$$

After a precipitate NaCl was filtered using a filter paper, all the solvent was removed in a vacuum at 60°C.

In order to obtain a solution in which the $HC(NH)(NH_2)$ prepared by the above-described process was dissolved in ethanol, the $HC(NH)(NH_2)$ was dissolved again in ethanol in a state where all the solvent was removed, and then, the process of filtering a precipitate NaCl using a filter paper was performed once again.

Then, HI was reacted with the $HC(NH)(NH_2)$, so that $HC(NH_2)_2I$ in the form of yellow powder as shown in **FIG. 2B** was prepared with a yield of from 10% to 20%.

**[Comparative Example 2]**

Preparation of precursor $HC(NH_2)_2I$

A precursor $HC(NH_2)_2I$ in the form of white powder as shown in **FIG. 2C** was prepared with a yield of 80% using $HC(NH)(NH_2)$, HI, and acetic acid as shown in the following Formula 5.

$$HC(NH_2)(NH). \; CH_3COOH + HI \rightarrow HC(NH_2)_2I + CH_3COOH \qquad (5)$$

A Perovskite solar cell was prepared in the same manner as Example using the $HC(NH_2)_2I$ prepared according to the synthesis method of Comparative Example 2.

Analysis of material properties

A $^1H$ nuclear magnetic resonance (NMR) spectrum was recorded on a Varian Unity Inova 300 MHz spectrometer using a chemical shift relative to a solvent peak as an internal reference of $^1H$ NMR dimethyl sulfoxide (DMSO, $\delta$ 2.5 ppm).

The high resolution mass spectroscopy (HRMS) was recorded according to a fast atom bombardment (FAB) method using a JEOL JMS-700 MStation.

An absorption coefficient was measured according to a conventional method.

In order to measure a transmittance and a reflectance, a $HC(NH_2)_2PbI_3$ film was used on a dense $TiO_2$-coated FTO glass.

For comparison, an absorption coefficient of a $CH_3NH_3PbI_3$ film was also checked.

The transmission and the reflectance were measured by a UV-vis spectrophotometer (Perkin Elmer, lamda 35) using an integrating sphere and a dense $TiO_2$-coated FTO glass as a blank for measurement of transmittance.

While a light was incident into the FTO glass in order to measure an absorbance, a light was incident into the Perovskite film in order to measure an absorption coefficient.

X-ray diffraction patterns were collected by a Bruker AXS (D8 advance, Bruker Corporation) using Cu Ka radiation at a scan rate of 4°/min.

Morphology of surface and cross section was obtained by a scanning electron microscope (SEM, JSM-7600F, JEOL).

Heat flow depending on a temperature was measured using a differential scanning calorimeter (DSC, Seico Inst., DSC7020 model).

Analysis of photovoltaic properties

A current and a voltage were measured using a Keithley 2400 source meter under one sun illumination (AM 1.5G, 100 mW/cm$^2$) and simulated by a solar simulator (Oriel Sol3A class AAA) equipped with a 450 W Xenon lamp (Newport 6279NS).

The intensity of light was regulated by a NREL-calibrated Si solar cell equipped with a KG-2 filter.

During the measurement, the device was covered with a metal aperture mask.

A photon-to-electron conversion efficiency (IPCE) was measured by a specially designed IPCE system (PV measurement Inc.).

A monochromatic beam was generated from a 75 W Xenon lamp (USHIO, Japan).

IPCE data were collected in a DC mode without bias light.

Time-limited photocurrent response was monitored using a 550 nm monochromatic beam at 4 Hz.

Property comparison between Example and Comparative Examples 1 and 2

In the precursor $HC(NH_2)_2I$ in accordance with Example, a broad $NH_2$ peak and a sharp CH peak were observed around 8.5 and 7.8 as shown in **FIG. 3,** and the other peaks were dimethylsulfoxide (hereinafter, also referred to as "DMSO") used as a solvent for the NMR measurement and water present in the solvent.

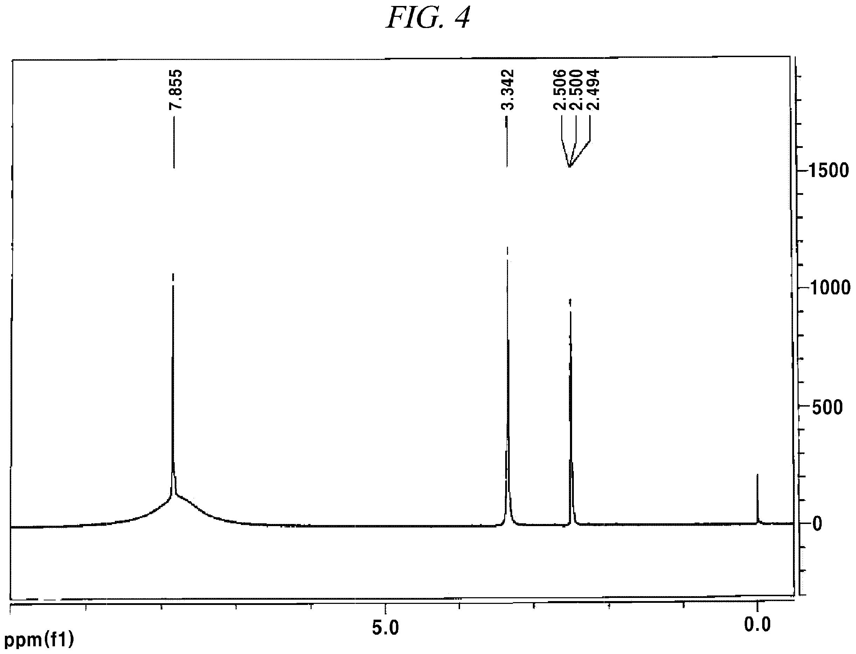

In the precursor $HC(NH_2)_2I$ in accordance with Comparative Example 1, a broad $NH_2$ peak and a sharp CH peak were observed around 7.855 as shown in **FIG. 4,** and the two peaks were overlapped. The other peaks were DMSO used as a solvent for the NMR measurement and water present in the solvent. Although not detected in the NMR spectrum, it could be seen that the precursor powder $HC(NH_2)_2I$ was remarkably different in color from those of Example and Comparative Example 2. In case of preparing a Perovskite solar cell using the precursor $HC(NH_2)_2I$ synthesized in accordance with Comparative Example 2, a Perovskite structure was not well formed, and, thus, it was difficult to evaluate properties as a device, and it was considered that this was because while the methanol was evaporated in preparing the precursor $HC(NH_2)_2I$, a reactant was also evaporated and the reactant reacted with NaCl.

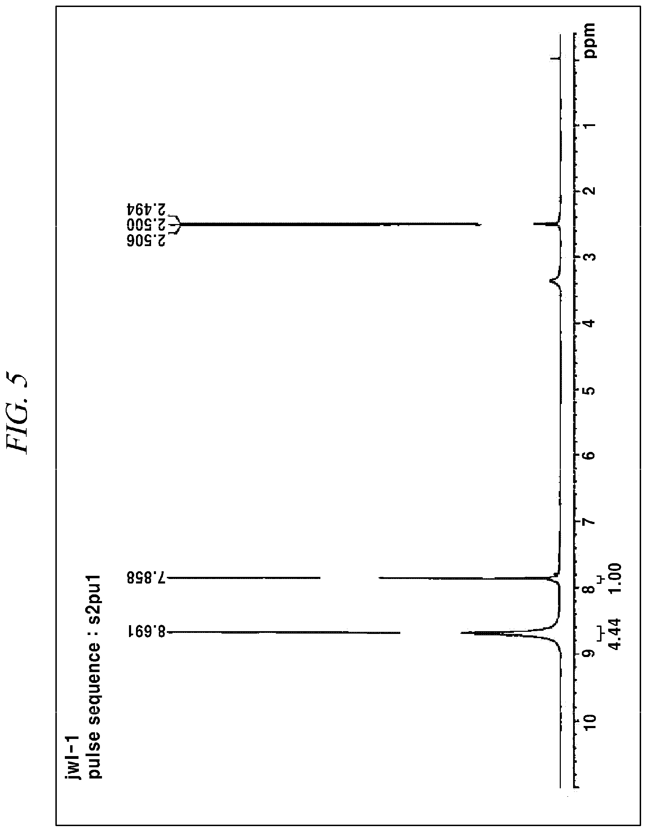

In the precursor $HC(NH_2)_2I$ in accordance with Comparative Example 2, a broad $NH_2$ peak and a sharp CH peak were observed around 9 and 7.855 as shown in **FIG. 5,** and the other peaks were DMSO used as a solvent for the NMR measurement and water present in the solvent.

Comparison in performance of $HC(NH_2)PbI_3$ solar cell depending on synthesis method of precursor $HC(NH_2)_2I$

The energy conversion efficiency of a Perovskite solar cell prepared using the precursor $HC(NH_2)_2I$ in accordance with Example and a Perovskite solar cell prepared using the precursor $HC(NH_2)_2I$ in accordance with Comparative Example 2 was checked.

Photocurrent density ($J_{SC}$), photovoltage ($V_{OC}$), fill factor (FF), and efficiency of the Perovskite solar cell prepared in accordance with Example were measured 13 times, and the results thereof were as listed in the following Table 1:

[Table 1]

| Example | $J_{SC}$(mA/cm$^2$) | $V_{OC}$ (V) | FF | Efficiency (%) |
|---|---|---|---|---|
| 1 | 19.43 | 1.055 | 0.71 | 14.52 |
| 2 | 19.47 | 1.055 | 0.71 | 14.59 |
| 3 | 18.77 | 1.047 | 0.7 | 13.79 |
| 4 | 19.05 | 1.038 | 0.67 | 13.26 |
| 5 | 19.03 | 1.048 | 0.66 | 13.22 |
| 6 | 18.93 | 1.038 | 0.65 | 12.83 |
| 7 | 19.27 | 1.051 | 0.67 | 13.55 |
| 8 | 19.12 | 1.064 | 0.67 | 13.61 |
| 9 | 18.49 | 1.058 | 0.68 | 13.29 |
| 10 | 19.23 | 1.029 | 0.66 | 13.08 |
| 11 | 19.37 | 1.043 | 0.71 | 14.25 |
| 12 | 19.48 | 1.033 | 0.71 | 14.28 |
| 13 | 19.72 | 1.034 | 0.68 | 13.79 |

(continued)

| Example | $J_{SC}$(mA/cm$^2$) | $V_{OC}$ (V) | FF | Efficiency (%) |
|---|---|---|---|---|
| Average | 19.18 | 1.05 | 0.68 | 13.7 |
| Standard deviation | 0.33 | 0.01 | 0.02 | 0.57 |

Photocurrent density ($J_{SC}$), photovoltage ($V_{OC}$), fill factor (FF), and efficiency of the Perovskite solar cell prepared in accordance with Comparative Example 2 were measured 11 times, and the results thereof were as listed in the following Table 2:

[Table 2]

| Comparative Example 2 | $J_{SC}$(mA/cm$^2$) | $V_{OC}$ (V) | FF | Efficiency (%) |
|---|---|---|---|---|
| 1 | 17.94 | 0.988 | 0.68 | 11.97 |
| 2 | 17.91 | 0.973 | 0.69 | 12.04 |
| 3 | 18.14 | 0.963 | 0.72 | 12.53 |
| 4 | 17.74 | 1.016 | 0.65 | 11.72 |
| 5 | 17.86 | 1.005 | 0.66 | 11.82 |
| 6 | 17.93 | 1.01 | 0.64 | 11.67 |
| 7 | 17.97 | 1.006 | 0.64 | 11.58 |
| 8 | 17.18 | 1.031 | 0.63 | 11.18 |
| 9 | 17.14 | 1.035 | 0.65 | 11.51 |
| 10 | 18.99 | 0.981 | 0.64 | 11.94 |
| 11 | 18.91 | 0.992 | 0.66 | 12.44 |
| Average | 17.97 | 1 | 0.66 | 11.85 |
| Standard deviation | 0.58 | 0.02 | 0.03 | 0.39 |

By comparison between Table 1 and Table 2, it could be seen that the efficiency of the Perovskite solar cell in accordance with Example was superior. Although not detected in the NMR spectrum of **FIG. 5,** acetic acid chemically bonded to the precursor $HC(NH_2)_2I$ could remain in case of using Comparative Example 2, and it was considered that this was because when the precursor $HC(NH_2)_2I$ was prepared in Comparative Example 2, acetic acid generated as a by-product had the boiling point of 118°C and had the same polarity as the precursor $HC(NH_2)_2I$, and, thus, it was not easy to completely remove the acetic acid.

[Verification of properties of Perovskite solar cell including precursor $HC(NH_2)_2I$ prepared in accordance with Example]

Absorption by photoactive layer

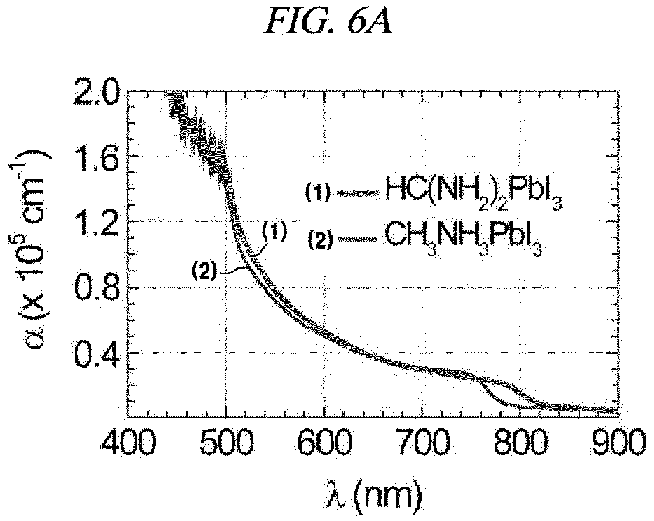

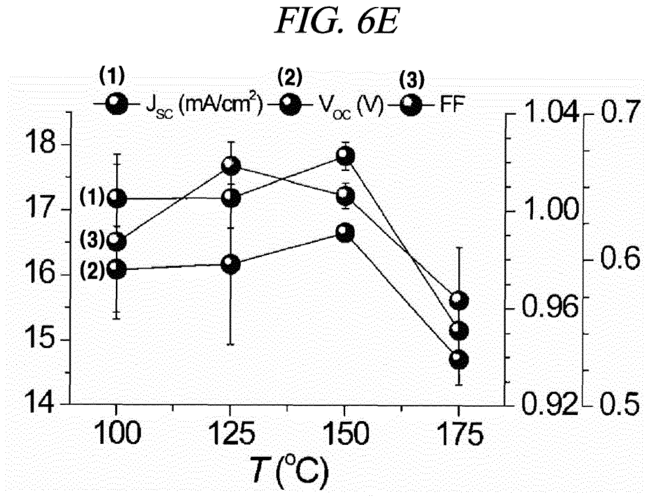

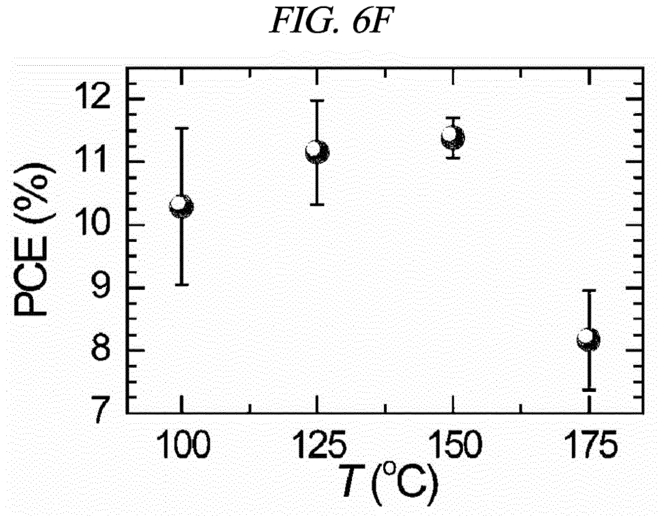

The absorption coefficient of a 250 nm-$HC(NH_2)_2PbI_3$ (hereinafter, also referred to as "FAPbI$_3$") film prepared in accordance with the present Example was 1.53 x 10$^5$ cm$^{-1}$ at 500 nm, 0.53 $\times$ 10$^5$ cm$^{-1}$ at 600 nm, and 0.30 x 10$^5$ cm$^{-1}$ at 700 nm (**FIG. 6A),** and measured using a formula reported in other studies.

The absorption coefficient of the FAPbI$_3$ at a given wavelength was slightly higher than that of $CH_3NH_3PbI_3$ (hereinafter, also referred to as "MAPbI$_3$") which has been conventionally studied.

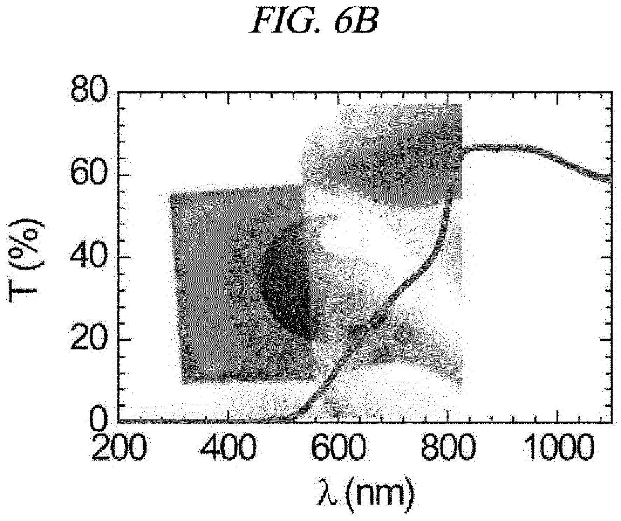

**A** FAPbI$_3$-coated 200 nm-$TiO_2$ film (total thickness: about 300 nm) was transparent brown as shown in **FIG. 6B.**

The overall transmittance after 840 nm showed that band gap absorption corresponding to energy of about 1.47 eV occurred around 840 nm, which is identical to the previous observation.

An increase in transmittance from 600 nm to 840 nm implies loss of light reflection at a long wavelength.

Annealing temperature dependence of photoactive layer

A photocurrent-voltage characteristic depending on a heat treatment temperature for the porous semiconductor/Perovskite complex layer of the Perovskite solar cell including the precursor $HC(NH_2)_2I$ prepared in accordance with the present Example and an external quantum efficiency corresponding to each device were measured.

The annealing temperature dependence of the deposited $FAPbI_3$ layer as a photovoltaic performance was checked (**FIG. 6A** through **FIG. 6F).**

Table 3 shows photocurrent density (Jsc), photovoltage (Voc), fill factor (FF), and photo-to-electron conversion efficiency of the Perovskite $HC(NH_2)_2PbI_3$ solar cell depending on a heat treatment temperature. The efficiency was observed to be highest in case of performing a heat treatment at 150°C, which was considered as being caused by a high current density resulting from a high quantum conversion efficiency.

[Table 3]

| Temperature | $J_{SC}(mA/cm^2)$ | $V_{OC}$ (V) | FF | Efficiency (%) |
|---|---|---|---|---|
| 100°C | 17.21 | 0.989 | 0.66 | 11.2 |
| 125°C | 17.35 | 0.996 | 0.66 | 11.32 |
| 150°C | 17.96 | 0.991 | 0.64 | 11.47 |
| 175°C | 15.73 | 0.943 | 0.55 | 8.17 |

The most superior PCE was obtained at 150°C mainly because of the highest photocurrent density ($J_{SC}$) and photovoltage ($V_{OC}$).

The annealing temperature dependence of $J_{SC}$ has a tendency similar to a change in $V_{OC}$ depending on a temperature, which seems to be probably caused by a relationship $V_{oc} = (k_BT/q)\ln(J_{SC}/J_0 + 1)$.

Accordingly, the PCE is increased along an increase in annealing temperature from 100°C to 150°C and then sharply decreased at 175°C.

The measurement of X-ray diffraction was conducted in order to find out the basis for strong dependence of the photovoltaic performance on an annealing temperature.

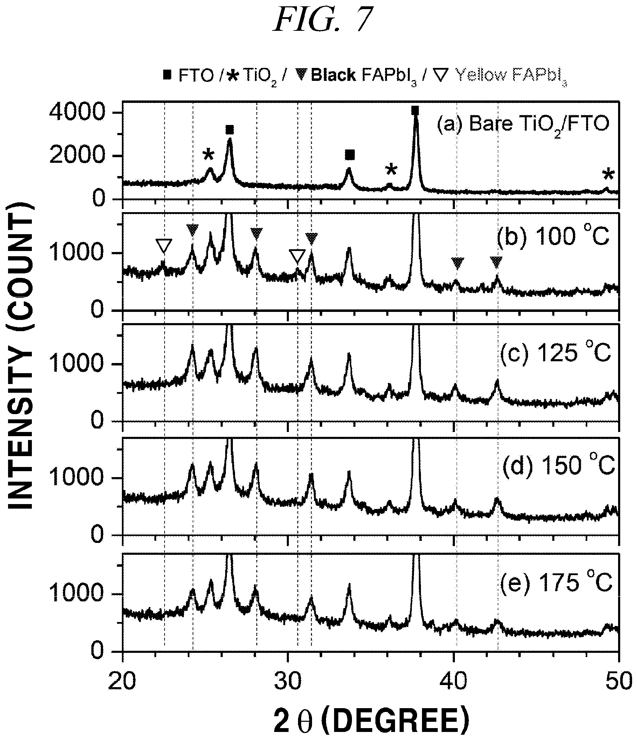

XRD reflection at 24.26°, 28.04°, 31.42°, 40.1°, and 42.7° (**FIG. 7A** through **FIG. 7E)** was indexed to (202), (220), (222), (400), and (330) reflections each corresponding to a black $FAPbI_3$ polymorph.

In a sample annealed at 100°C, a yellow polymorph coexisted with a black polymorph and disappeared in a higher annealing temperature condition.

According to all of data from experiments and simulations using black $FAPbI_3$, the (220) and (222) reflections were stronger than the (202) reflection.

In a sample in accordance with the present Example, the intensity of the (202) reflection was almost similar to those of the (202) and (222) reflections, which shows that preferred orientation of the $FAPbI_3$ layer of the present disclosure is different from the reported result.

Decomposition of the $FAPbI_3$ was not observed in the temperature range of from 100°C to 175°C, which is connected to no heat transfer in the above-described temperature range measured using a differential scanning calorimeter (DSC) (of which data are not illustrated).

In connection with decomposition of the $FAPbI_3$, color bleaching was observed in a sample heat-treated at 200°C.

**An** effect of a duration at a given annealing temperature on the photovoltaic performance was checked.

When a duration at 150°C was increased from 10 minutes to 15 minutes, $J_{SC}$ was slightly increased with almost no change in $V_{OC}$ and fill factor (FF).

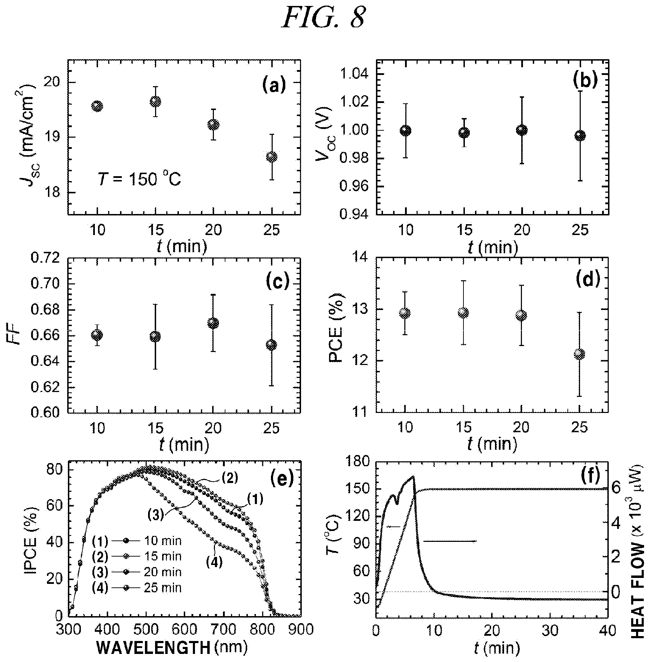

Accordingly, the PCE was slightly higher in the 15 min-heat treatment (**FIG. 8A** through **FIG.8F).**

However, the PCE was decreased when the duration was increased from 15 minutes to 20 minutes and 25 minutes, and this is because of a decrease in $J_{SC}$ connected to IPCE which is usually low at a long wavelength.

The deterioration of performance in a long time-heat treatment at 150°C is connected to partial decomposition of the $FAPbI_3$ as indicated by a negative heat flow in a DSC analysis (**FIG. 8F).**

A heating condition for DSC measurement is not exactly identical to that for preparation of a Perovskite sample, but it is clear that crystallization is completed within 15 minutes.

The most superior performance was obtained from the heat treatment of the $FAPbI_3$ at 150°C, and, thus, parameters were further optimized for 150°C-15 min-annealed $FAPbI_3$.

Effect of porous $TiO_2$ film on morphology of photoactive layer

As the effects of a porous $TiO_2$ film on the morphology of the deposited $FAPbI_3$, charge collection kinetics and photovoltaic performance were checked.

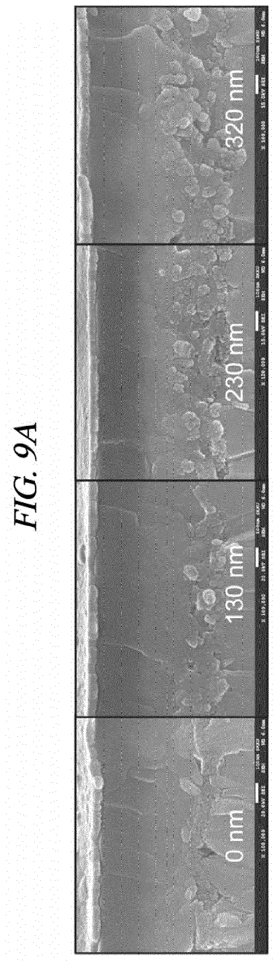

**As** shown in **FIG. 9A,** $FAPbI_3$ having a thickness of about 300 nm was formed on a dense wall layer, and $FAPbI_3$ was formed to a similar thickness on 130 nm- and 230 nm-porous $TiO_2$ films, but $FAPbI_3$ was formed to a slightly increased thickness on a 320 nm-$TiO_2$ film.

The pores of the porous $TiO_2$ films were entirely filled with $FAPbI_3$, and upper parts of the deposited $FAPbI_3$ layers appear flat.

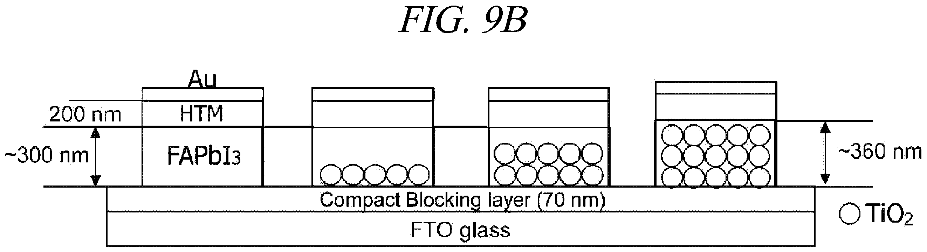

**FIG. 9B** schematically illustrates structures of the layers.

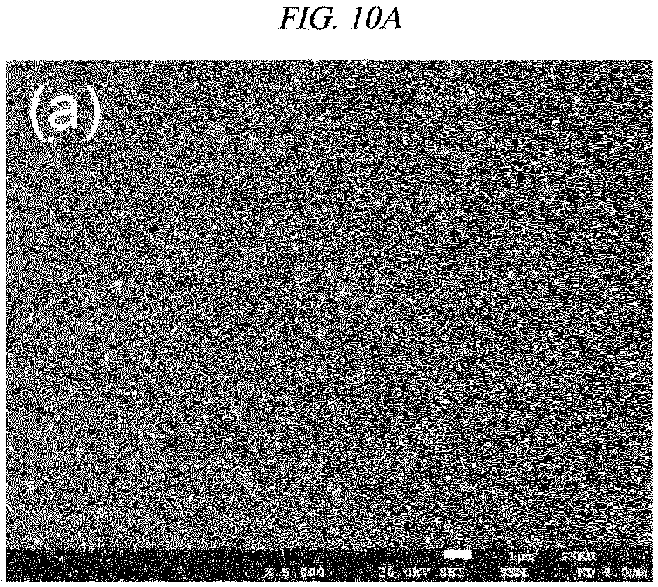

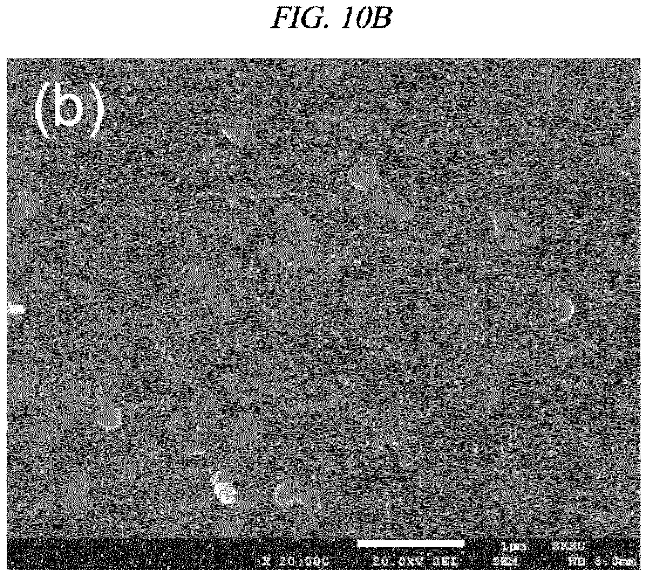

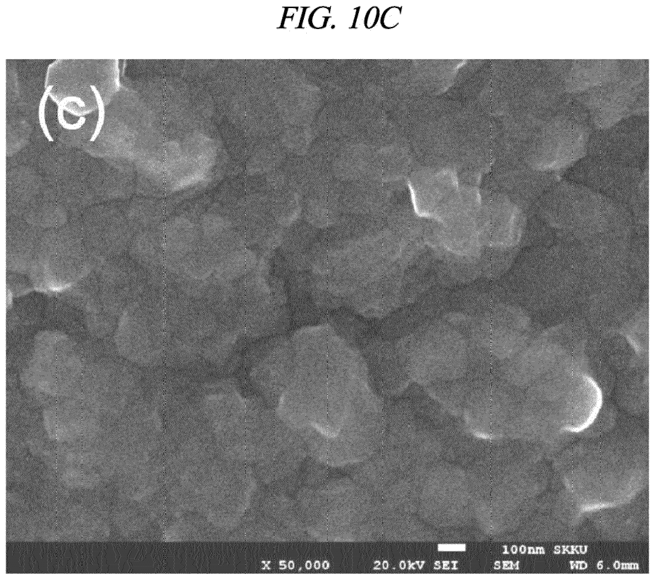

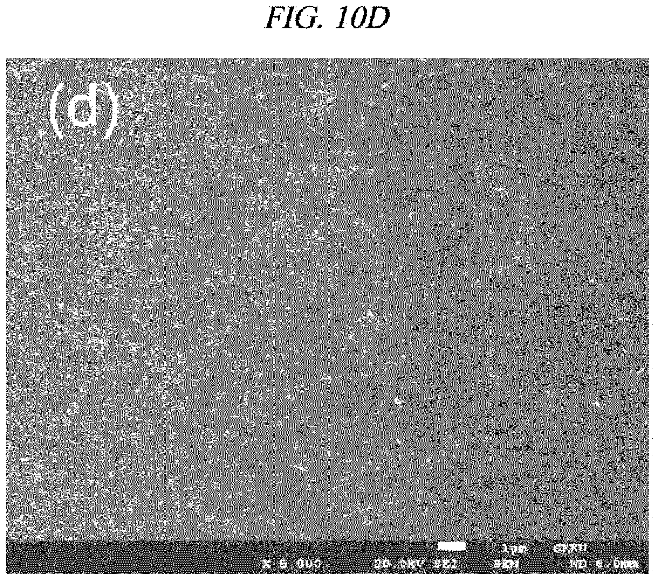

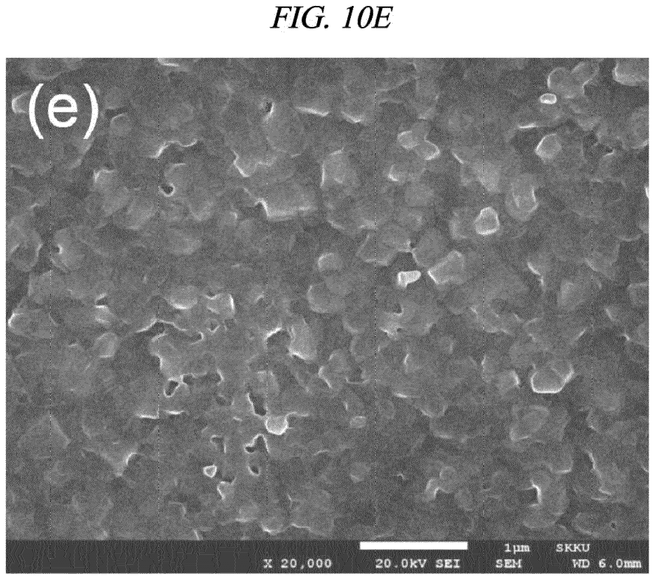

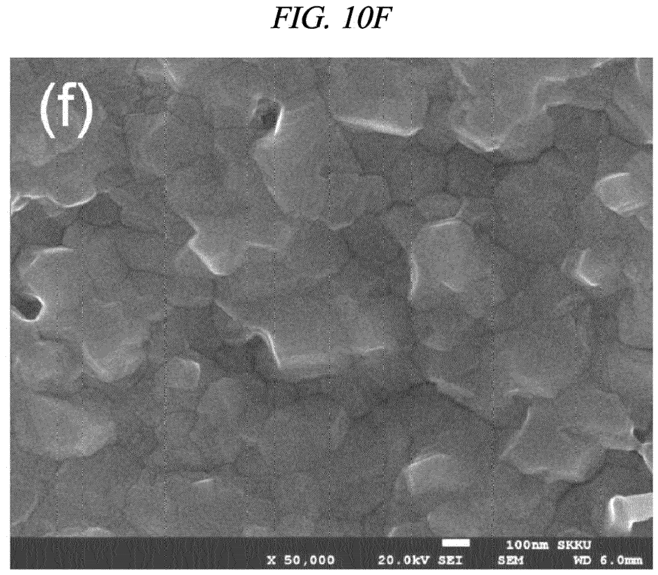

A surface coverage of $FAPbI_3$ coated on a dense $TiO_2$ layer without a porous $TiO_2$ layer (planar structure) or coated on a porous $TiO_2$ layer (mesoscopic structure) was checked in the SEM images of the upper parts (**FIG. 10A** through **FIG. 10F**). It was confirmed that these two structures were entirely filled with the $FAPbI_3$.

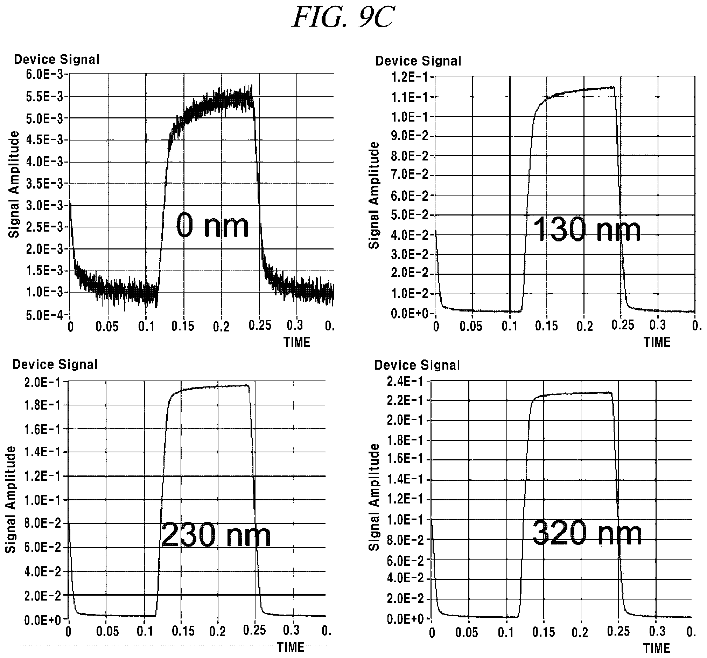

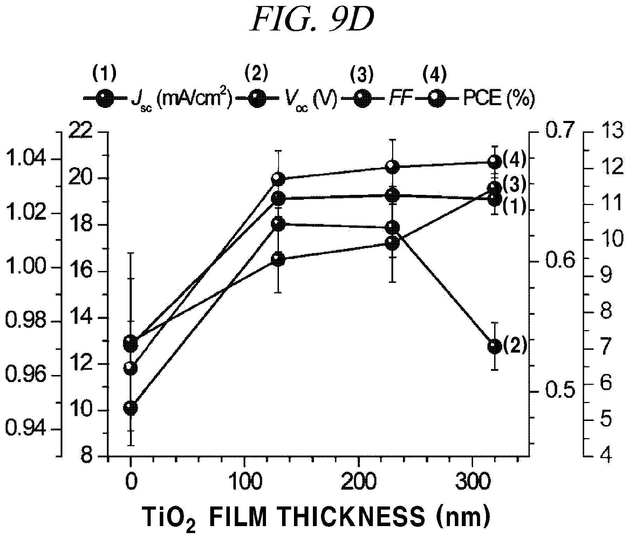

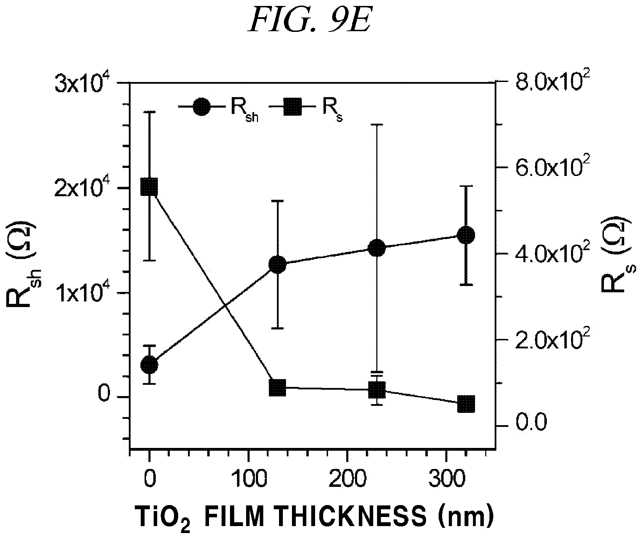

The presence and absence of a porous $TiO_2$ film and a thickness of the $TiO_2$ film considerably affect a charge collection rate (**FIG. 9C**), photovoltaic performance (**FIG. 9D**), shunt, and series resistances (**FIG. 9E**).

As shown in **FIG. 9C**, a time-limited photocurrent signal was detected under 550 nm-monochromatic beam.

A low amplitude of the device signal, associated with charge collection, and slow kinetics were observed in the $FAPbI_3$ Perovskite solar cell without a porous $TiO_2$ film.

Meanwhile, the device signal amplitude was increased with an increase in thickness of a $TiO_2$ film, along with a fast collection rate.

The presence of a porous $TiO_2$ film is important in facilitating photocurrent collection.

$J_{SC}$ was significantly improved from about 13 mA/cm$^2$ to about 19 mA/cm$^2$ by introduction of the porous $TiO_2$ film.

$V_{OC}$ was also improved from 948 mV to 1,015 mV, but slightly decreased to 971 mV in the 320 nm-$TiO_2$ film.

The slight decrease of $V_{OC}$ in the 320 nm-$TiO_2$ film may be probably connected to recombination caused by the increased $FAPbI_3$ layer.

As compared with the device without a porous $TiO_2$ film, FF is increased when a porous $TiO_2$ film is included and as its thickness is increased.

A series resistance is decreased by more than one order of magnitude and a shunt resistance is increased by 4.6 times (**FIG. 9E**), which results from the improved fill factor.

A high series resistance and a low shunt resistance in a $FAPbI_3$ layer without a porous $TiO_2$ film indicates the presence of a resistance-related component.

An abrupt decrease in series resistance and increase in shunt resistance by including a porous $TiO_2$ film implies that underlying resistance components are removed by the presence of the $TiO_2$ film probably as a result of charge separation at the $TiO_2/FAPbI_3$ interface.

It may be deduced that the origin of shunt resistance is inferred from the area uncovered with One can argue that the origin of shunt resistance may be inferred from the area uncovered with $FAPbI_3$ associated with leakage paths, which is however ruled out since the full coverage of $FAPbI_3$ is found both in planar and mesoporous structures, as shown in **FIG. 10A** through **FIG. 10F**.

An abrupt decrease of shunt resistance upon removal of a porous $TiO_2$ layer obviously indicates that the porous $TiO_2$ layer is important in protecting shunting current.

The function of the porous $TiO_2$ layer is clear as demonstrated by a gradual increase of shunt resistance along with an increase of a $TiO_2$ layer.

Detection of IPCE is also affected by the presence and thickness of a $TiO_2$ film.

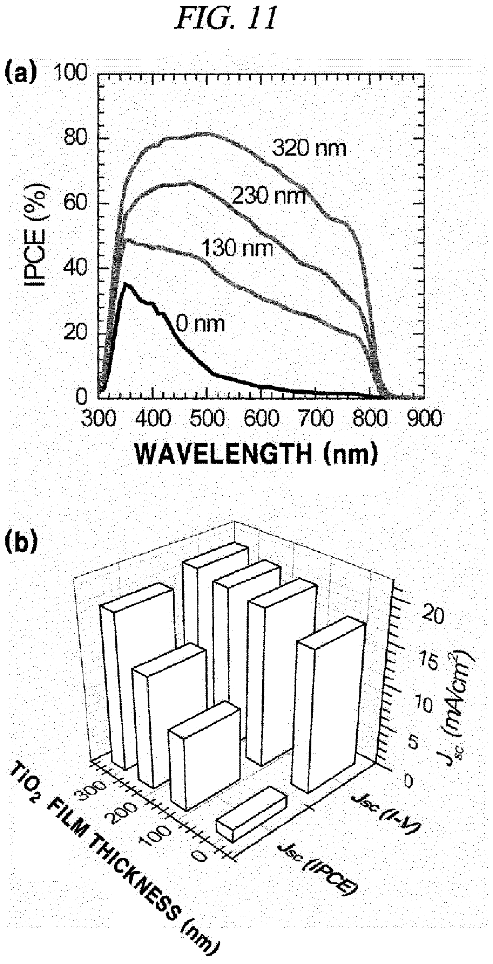

While a large discrepancy was observed with a decrease in thickness of a $TiO_2$ film (**FIG. 11A** and **FIG. 11B**), integrated $J_{SC}$ (18.9 mA/cm$^2$) calculated from ICPE data was almost identical to $J_{SC}$ (20.2 mA/cm$^2$) obtained from a sun simulation with respect to the 320 nm-$TiO_2$ film.

The improvement in IPCE signal by including and increasing a porous $TiO_2$ layer indirectly proves injection of electrons into a $TiO_2$ layer, which can be explained partially as a basis for resistance change.

Therefore, the presence of a porous $TiO_2$ layer is advantageous as compared with a planar structure in terms of resistance control and charge collection.

Further, the excellent photovoltaic performance of a porous $TiO_2$ solar cell is connected to an unbalanced electron-hole diffusion length of $FAPbI_3$.

In a methylammonium lead iodide Perovskite, it was considered that as for a $FAPbI_3$ layer, an uneven surface was more suitable for electron and hole diffusion and charge separation than an even surface on the basis of a balanced electron and hole transport characteristic.

To this end, in the present disclosure, a mixed porous $TiO_2$ nanostructure including small and large pores by means of variation of content of polymer vehicle ethyl cellulose (EC) in $TiO_2$ paste was designed.

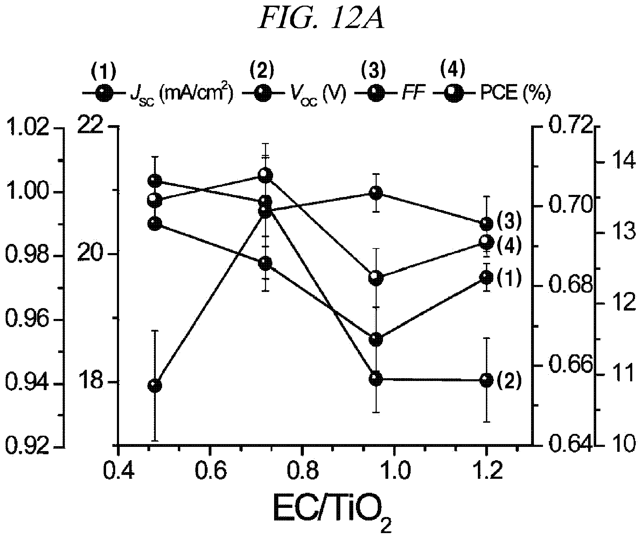

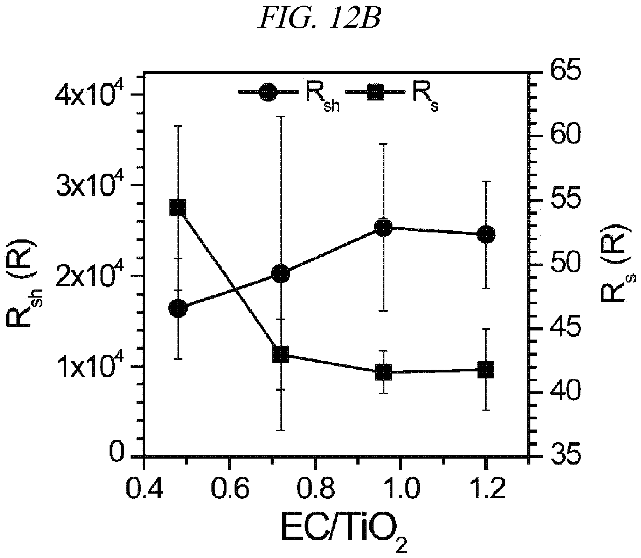

A significant improvement in FF was achieved by increasing EC content from EC/$TiO_2$ = 0.48 to 0.72 (**FIG. 12A**), which is caused by a decrease in series resistance and an increase in shunt resistance (**FIG. 12B**).

The microporous structure formed by high EC content is expected to induce large $FAPbI_3$ crystals, which is likely to reduce the number of grain boundaries relating to resistance components.

As a consequence of improvement in FF, PCE was improved in spite of slightly decreased $J_{SC}$ and $V_{OC}$.

Too much EC content is not recommended because of the loss of $V_{OC}$.

An increase in amount of EC results in large pores (100 nm or more) together with small pores (about 50 nm).

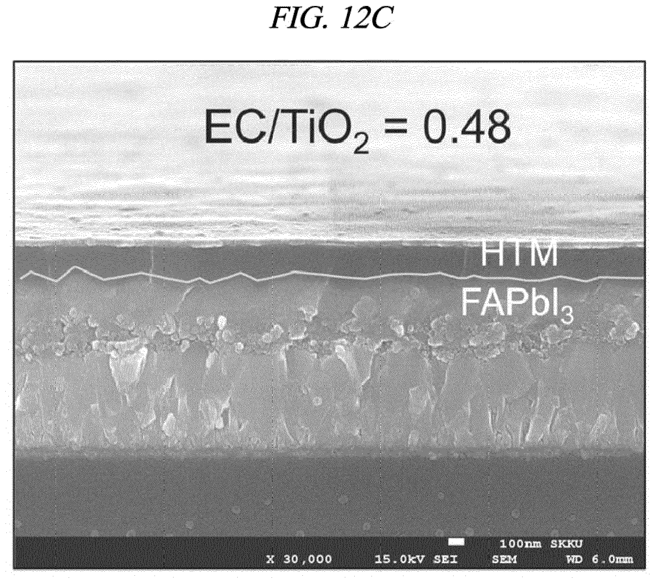

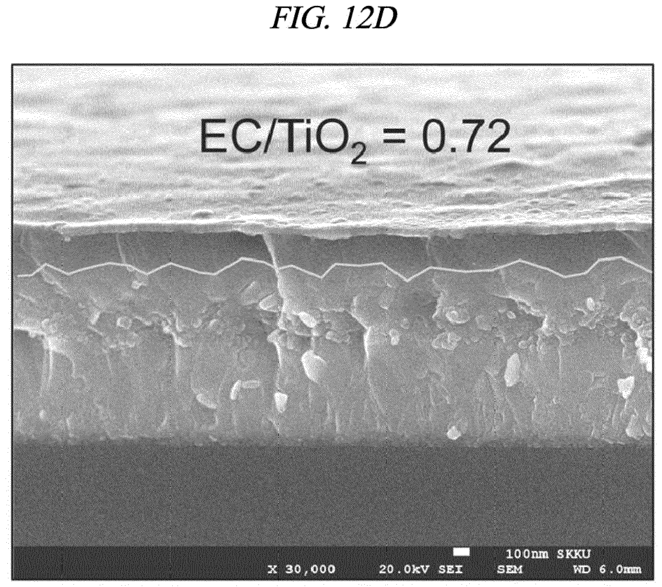

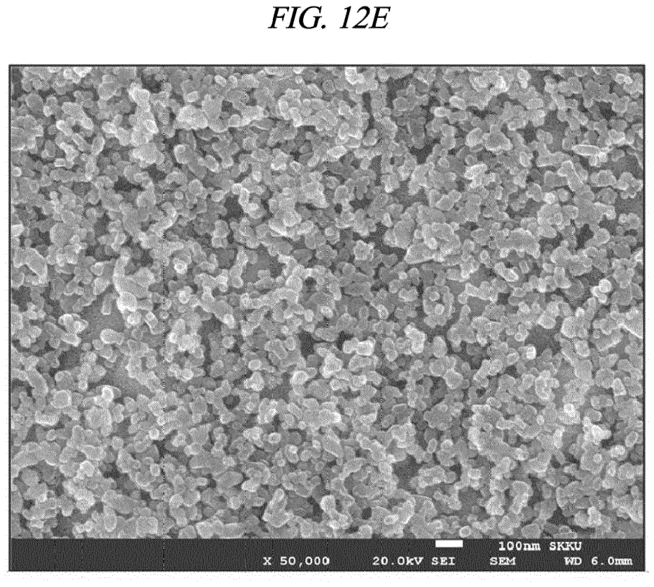

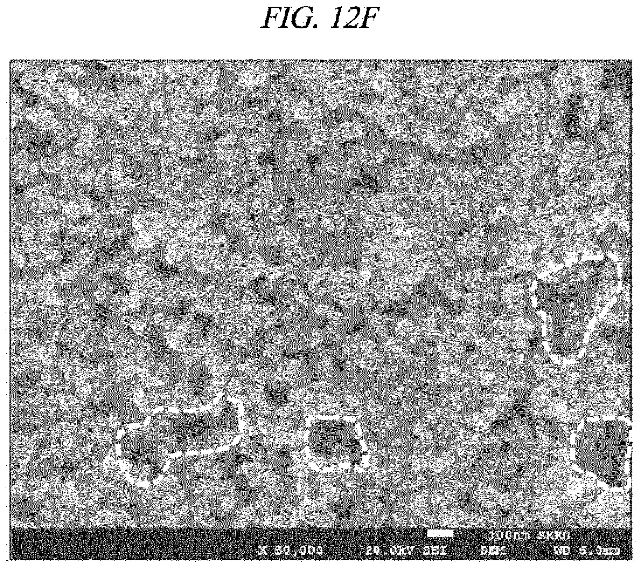

These mixed pores led to a $TiO_2$ film including large pores of about 300 nm along with addition of EC as shown in **FIG. 12D** and **FIG. 12F** and thus led to an uneven surface of FAPbI$_3$ as compared with a TiO$_2$ film including pores of from 40 nm to 100 nm (**FIG. 12C** and **FIG. 12E**).

From the systematic evaluation, it is clear that a meso-micro porous TiO$_2$ film is needed to decrease a series resistance and increase a shunt resistance in a FAPbI$_3$ Perovskite solar cell.

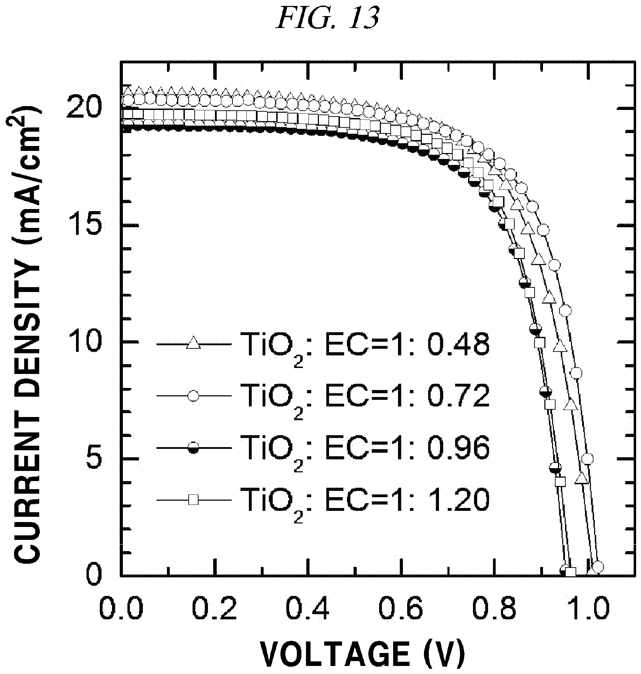

**FIG. 13** shows a current-voltage characteristic depending on a r composition ratio of a polymer in TiO$_2$ nanoparticle paste used for forming the porous TiO$_2$ nanoparticle layer, and it could be seen that when a ratio of the TiO$_2$ nanoparticles : polymer (ethyl cellulose) was 1:0.72, the highest efficiency was obtained, and also it could be seen that a fill factor (FF) was improved depending on a composition ratio of the polymer.

Table 4 shows photocurrent density (Jsc), photovoltage (Voc), fill factor (FF), and photo-to-electron conversion efficiency depending on a ratio of the polymer in the TiO$_2$ nanoparticle paste in preparing a HC(NH$_2$)$_2$PbI$_3$ Perovskite solar cell.

[Table 4]

| Content (TiO$_2$ : EC) | J$_{SC}$(mA/cm$^2$) | V$_{OC}$ (V) | FF | Efficiency (%) |
|---|---|---|---|---|
| 1:0.48 | 20.6 | 1.011 | 0.67 | 13.9 |
| 1:0.72 | 20.33 | 1.022 | 0.69 | 14.32 |
| 1:0.96 | 19.3 | 0.953 | 0.7 | 12.78 |
| 1:1.20 | 19.75 | 0.964 | 0.69 | 13.19 |

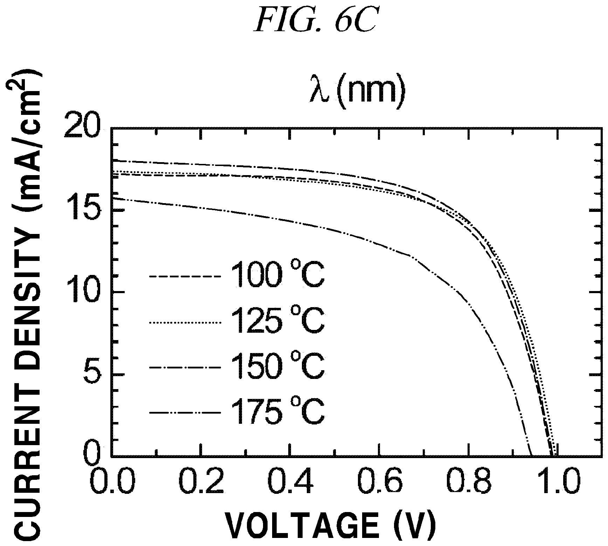

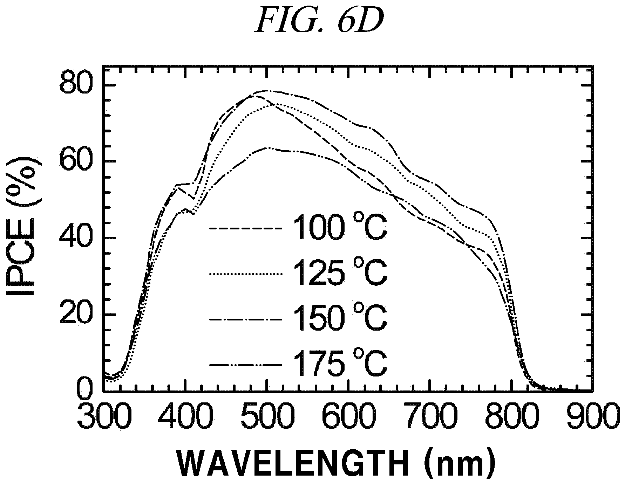

**FIG. 12C** and **FIG. 12D** are electron microscopic cross-sectional images depending on a composition ratio of a polymer in TiO$_2$ nanoparticle paste used for forming a porous TiO$_2$ nanoparticle layer, and it could be seen that as a composition ratio of the polymer was increased, an interface between a Perovskite layer and a hole transport layer was increased, which may contribute to the improvement in FF as shown in **FIG. 6C** and **FIG. 6D**.

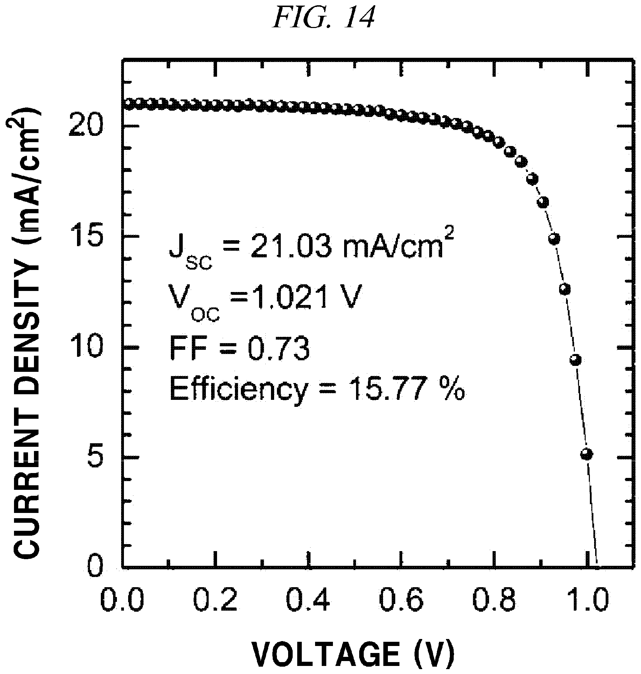

**FIG. 14** shows a photocurrent-voltage graph for a HC(NH$_2$)$_2$PbI$_3$ solar cell prepared using the most optimum conditions found through experiments, and photocurrent density (Jsc), photovoltage (V$_{OC}$), fill factor (FF), and photo-to-electron conversion efficiency of the HC(NH$_2$)$_2$PbI$_3$ solar cell prepared in these conditions were measured 6 times, and the results thereof were as listed in the following Table 5.

[Table 5]

| | J$_{SC}$(mA/cm$^2$) | V$_{OC}$ (V) | FF | Efficiency (%) |
|---|---|---|---|---|
| 1 | 21.03 | 1.021 | 0.73 | 15.77 |
| 2 | 20.35 | 1.017 | 0.72 | 14.87 |
| 3 | 20.45 | 1.013 | 0.71 | 14.7 |
| 4 | 22.34 | 0.985 | 0.68 | 15.04 |
| 5 | 20.75 | 1.007 | 0.73 | 15.26 |
| 6 | 20.7 | 1.013 | 0.73 | 15.36 |
| Average | 20.94 | 1.009 | 0.72 | 15.17 |
| Standard deviation | 0.66 | 0.012 | 0.02 | 0.35 |

It could be seen from **FIG. 14** and Table 5 that the HC(NH$_2$)$_2$PbI$_3$ solar cell having an average efficiency of 15.17% was reproducibly prepared.

To summarize, methylammonium lead iodide HC(NH$_2$)$_2$PbI$_3$-based high-efficiency, J-V hysteresis-free and photo-stable Perovskite solar cell was successfully prepared. HC(NH$_2$)$_2$PbI$_3$ was prepared by immersing PbI$_2$-coated electrode in HC(NH$_2$)$_2$I solution. It was found that the annealing temperature and time for the deposited HC(NH$_2$)$_2$PbI$_3$ is very important for photovoltaic performance. A black HC(NH$_2$)$_2$PbI$_3$ polymorph formed at 150°C for 15 minutes showed the highest photocurrent. It was also found that a porous TiO$_2$ layer is important for improvement in other parameters such as charge collection, fill factor, and voltage. A high fill factor was realized by engineering a porous structure of the TiO$_2$ layer via EC/TiO$_2$ ratio control.

The above description of the present disclosure is provided for the purpose of illustration, and it would be understood by those skilled in the art that various changes and modifications may be made without changing technical conception and essential features of the present disclosure. Thus, it is clear that the above-described examples are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims are included in the scope of the present disclosure.

**Claims**

1.  A method of preparing a precursor for producing a Perovskite, comprising:

    reacting formamidine·HCl with sodium ethoxide to obtain a formamidine; and
    reacting the obtained formamidine with HI to obtain formamidinium iodide.

2.  A method of preparing a Perovskite solar cell, comprising:

    forming a recombination-preventing layer on a first electrode including a transparent conductive substrate;
    forming a photoactive layer including a layer of a Perovskite by forming a layer of a Perovskite represented by the following Chemical Formula 1 on the recombination-preventing layer;
    forming a hole transport layer on the photoactive layer; and
    forming a second electrode on the hole transport layer;

    [Chemical Formula 1]          $RMX_3$,

    wherein the forming of a photoactive layer includes forming the layer of the Perovskite represented by Chemical Formula 1 which is formed by reacting $MX_2$ wherein M includes a +2 metal cation selected from the group consisting of $Pb^{2+}$, $Sn^{2+}$, $Ge^{2+}$, and combinations thereof and X is a halogen anion with R being the formamidinium cation of the precursor prepared by the method according to Claim 1.

3.  The method of preparing a Perovskite solar cell of Claim 2,
    wherein the photoactive layer further includes a semiconductor layer.

4.  The method of preparing a Perovskite solar cell of Claim 3,
    wherein the semiconductor layer has a porous structure including pores of from 20 nm to 500 nm.

5.  The method of preparing a Perovskite solar cell of Claim 4, further comprising:
    after the forming of the photoactive layer, annealing the photoactive layer at from 100°C to 170°C.

**Patentansprüche**

1.  Verfahren zur Herstellung eines Vorläufers für die Erzeugung eines Perowskits, welches umfasst:

    Umsetzen von Formamidin·HCl mit Natriumethoxid, um ein Formamidin zu erhalten; und
    Umsetzen des erhaltenen Formamidins mit HI, um Formamidiniumlodid zu erhalten.

2.  Verfahren zur Herstellung einer Perowskit-Solarzelle, welches umfasst:

    Bilden einer Rekombination verhindernden Schicht auf einer ersten Elektrode, die ein transparentes, leitfähiges Substrat enthält;
    Bilden einer photoaktiven Schicht, die eine Schicht eines Perowskits enthält, durch Bilden einer Schicht eines durch die folgende Chemische Formel 1 dargestellten Perowskits auf der Rekombination verhindernden Schicht;
    Bilden einer Lochtransportschicht auf der photoaktiven Schicht; und
    Bilden einer zweiten Elektrode auf der Lochtransportschicht;

    [Chemische Formel 1]          $RMX_3$,

wobei das Bilden einer photoaktiven Schicht das Bilden der Schicht des durch Chemische Formel 1 dargestellten Perowskits umfasst, der durch Umsetzung von $MX_2$ gebildet wird, wobei M ein +2-Metallkation enthält, das aus der Gruppe ausgewählt ist, welche aus $Pb^{2+}$, $Sn^{2+}$, $Ge^{2+}$ und Kombination derselben besteht, und X ein Halogen-Anion ist, wobei R das Formamidinium-Kation des durch das Verfahren gemäß Anspruch 1 hergestellten Vorläufers ist.

**3.** Verfahren zur Herstellung einer Perowskit-Solarzelle nach Anspruch 2, wobei die photoaktive Schicht zudem eine Halbleiterschicht enthält.

**4.** Verfahren zur Herstellung einer Perowskit-Solarzelle nach Anspruch 3,
wobei die Halbleiterschicht eine poröse Struktur aufweist, die Poren von 20 nm bis 500 nm enthält.

**5.** Verfahren zur Herstellung einer Perowskit-Solarzelle nach Anspruch 4, welches zudem umfasst:
nach dem Bilden der photoaktiven Schicht ein Tempern der photoaktiven Schicht bei 100°C bis 170°C.

**Revendications**

**1.** Procédé pour préparer un précurseur destiné à produire une pérovskite, comprenant les étapes consistant à :

faire réagir de la formamidine HCl avec de l'éthoxide de sodium pour obtenir une formamidine ; et
faire réagir la formamidine obtenue avec HI pour obtenir de l'iodure de formamidinium.

**2.** Procédé pour préparer une cellule solaire à pérovskite, comprenant les étapes consistant à :

former une couche d'empêchement de recombinaison sur une première électrode incluant un substrat conducteur transparent ;
former une couche photoactive incluant une couche constituée d'une pérovskite en formant une couche constituée d'une pérovskite représentée par la formule chimique 1 suivante sur la couche d'empêchement de recombinaison ;
former une couche de transport de trous sur la couche photoactive ; et
former une seconde électrode sur la couche de transport de trous ;

$$[\text{formule chimique 1}] \qquad RMX_3,$$

dans lequel l'étape consistant à former une couche photoactive inclut de former la couche constituée de la pérovskite représentée par la formule chimique 1 qui est formée en faisant réagir $MX_2$, où M inclut un cation de métal +2 sélectionné parmi le groupe constitué de : $Pb^{2+}$, $Sn^{2+}$, $Ge^{2+}$ et des combinaisons de ceux-ci, et X est un anion halogène avec R étant le cation de formadiminium du précurseur préparé par le procédé selon la revendication 1.

**3.** Procédé pour préparer une cellule solaire à pérovskite selon la revendication 2,
dans lequel la couche photoactive inclut en outre une couche semi-conductrice.

**4.** Procédé pour préparer une cellule solaire à pérovskite selon la revendication 3,
dans lequel la couche semi-conductrice a une structure poreuse incluant des pores allant de 20 nm à 500 nm.

**5.** Procédé pour préparer une cellule solaire à pérovskite selon la revendication 4, comprenant en outre l'étape consistant à :
après avoir formé la couche photoactive, recuire la couche photoactive à une température allant de 100 °C à 170 °C.

## FIG. 1A

## FIG. 1B

## FIG. 1C

## FIG. 2

FIG. 3

EP 3 135 633 B1

*FIG. 4*

*FIG. 5*

## FIG. 6A

## FIG. 6B

## FIG. 6C

## FIG. 6D

## FIG. 6E

## FIG. 6F

## FIG. 7

## FIG. 8

*FIG. 9A*

*FIG. 9B*

EP 3 135 633 B1

# FIG. 9C

EP 3 135 633 B1

## FIG. 9D

FIG. 9E

FIG. 10A

## FIG. 10B

FIG. 10C

*FIG. 10D*

## FIG. 10E

FIG. 10F

# FIG. 11

## FIG. 12A

*FIG. 12B*

## FIG. 12C

## FIG. 12D

*FIG. 12E*

*FIG. 12F*

## FIG. 13

## FIG. 14

$J_{SC}$ = 21.03 mA/cm$^2$
$V_{OC}$ =1.021 V
FF = 0.73
Efficiency = 15.77 %

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014045021 A1

**Non-patent literature cited in the description**

- *J. Am. Chem. Soc.,* 2009, vol. 131, 6050-6051
- *Sci. Rep.,* 2012, vol. 2 (579), 1-7
- **S. PANG et al.** *Chem. Mater.,* 2014, vol. 26, 1485-1491
- **G. E. EPERON et al.** *Energy Environ. Sci.,* 2014, vol. 7, 982-988
- **C. C. STOUMPOS et al.** *Inorg. Chem.,* 2013, vol. 52, 9019-9038
- *Inorg. Chem.,* 2013, vol. 52, 9019-9038
- *Energy Environ. Sci.,* 2014, vol. 7, 982-988